# EUROPEAN PATENT APPLICATION

(11) **EP 4 272 617 A1**
(43) Date of publication of application: **08.11.2023**
(21) Application number: 22197810.9
(22) Date of filing: 26.09.2022
(51) Int. Cl.: A47L 11/04, A47L 11/20, A47L 11/30, A47L 11/40

(54) **ROBOTIC SYSTEMS AND METHODS**

(30) Priority: 05.05.2022 WO PCT/CN2022/091020; 13.07.2022 WO PCT/CN2022/105467
(71) Applicant: Intelligent Cleaning Equipment Holdings Co., Ltd., Tortola (VG)
(72) Inventor: CHEN, Simon Nai Pong, Guangdong, 523400 (CN); ABELLI, Stefano Daniele, 29122 Piacenza PC (IT); GHIRARDUZZI, Gianfranco, 29122 Piacenza (IT); YANG, Lianhua, Guangdong, 523400 (CN)
(74) Representative: Clark, Jane Anne

(57) **Abstract**

The present disclosure provides robotic systems and methods. The robotic systems and methods may be used to clean an area or environment. In embodiments a floor cleaning machine comprises: a mobile body configured to travel over a surface with aid of a drive mechanism; one or more cleaning devices coupled to the mobile body and configured to clean the surface by collecting and removing foreign materials from the surface; and a power source carried by the mobile body and configured to power the drive mechanism and the one or more cleaning devices, wherein the power source is configured to enable the floor cleaning machine to operate for a duration ranging from about 0.5 to about 4 hours, wherein the floor cleaning machine has a volume ranging from about 0.05 to about 0.30 cubic meters (m³).

## Description

### CROSS REFERENCE

This application claims priority to International Applications Nos. PCT/CN2022/091020 filed on May 5, 2022 and PCT/CN2022/105467 filed on July 13, 2022, which applications are incorporated herein by reference in their entirety for all purposes.

### BACKGROUND

Robots and/or machines may be used to perform tasks, provide services, and navigate environments autonomously or semi-autonomously. Some robots or machines may be used to execute cleaning tasks in an environment in which the robot or machine is positioned or capable of navigating to or through with or without input or command by a human operator.

### SUMMARY

The present disclosure relates generally to robotic systems and methods. The robotic systems may comprise robots or machines that are capable of cleaning, disinfecting, or sanitizing an area or an environment. The robots or machines may be configured to operate autonomously or semi-autonomously to clean an area or environment.

The present disclosure addresses various limitations and shortcomings of conventional robots and machines in the cleaning space. Commercially available robots and machines are unable to operate efficiently in certain environments due to their size, shape, and/or configuration of components. Certain tradeoffs made when designing the robots or machines can significantly impact the ability of a robot or machine to efficiently execute a cleaning route, a cleaning plan, or a cleaning routine. For example, small household robots may not be able to clean large areas efficiently, and may not have the battery capacity for industrial or commercial cleaning tasks. As another example, larger robots for commercial cleaning may not have the maneuverability to navigate tighter spaces, and cannot clean difficult to reach spots due to their size / footprint.

The present disclosure provides robotic systems that are compact in size, highly maneuverable, efficient and productive, and powerful enough for commercial cleaning operations in environments spanning up to 15,000 square feet or more. The form factors of the robotic systems allow the robotic systems to clean in and around tight areas precisely without colliding with obstacles, and the selection and configuration of components allows the robotic systems to clean large areas or volumes efficiently. The combination of form factor and cleaning performance as exemplified in the various embodiments described herein enables both meticulous cleaning of hard to reach spots and high productivity rates / area coverage. The robotic systems disclosed herein provide an optimal balance between size and cleaning performance, and can be configured to clean many different types of environments thoroughly and effectively.

In one aspect, the present disclosure provides a floor cleaning machine. In some embodiments, the floor cleaning machine may comprise a mobile body configured to travel over a surface with aid of a drive mechanism. In some embodiments, the floor cleaning machine may comprise one or more cleaning devices coupled to the mobile body and configured to clean the surface by collecting and removing foreign materials from the surface. In some embodiments, the floor cleaning machine may comprise a power source carried by the mobile body and configured to power the drive mechanism and the one or more cleaning devices.

In some embodiments, the power source is configured to enable the floor cleaning machine to operate for a duration ranging from about 0.5 to about 4 hours. In some embodiments, the floor cleaning machine has a volume ranging from about 0.05 to about 0.30 cubic meters (m³). In some embodiments, the floor cleaning machine has a lateral footprint ranging from about 0.10 to 0.40 square meter (m²). In some embodiments, the floor cleaning machine has a weight ranging from about 30 to about 80 kg. In some embodiments, the drive mechanism is configured to enable a minimum turning radius during cleaning operations of about 500 mm to about 800 mm for the floor cleaning machine. In some embodiments, the drive mechanism is configured to enable the floor cleaning machine to move at a speed of up to about 3.6 km/hour. In some embodiments, the floor cleaning machine is configured to have a cleaning surface productivity rate ranging from about 100 to about 2000 m²/hour. In some embodiments, the floor cleaning machine is capable of operating at a flow rate ranging from 0 mL/min to about 300 mL/min.

In some embodiments, the power source comprises a battery. In some embodiments, the battery has a capacity ranging from about 200 Watt-hours to about 900 Watt-hours. In some embodiments, the power source comprises a secondary battery. In some embodiments, the secondary battery is detachable from the mobile body. In some embodiments, the secondary battery has a capacity ranging from about 200 Watt-hours to about 900 Watt-hours.

In some embodiments, the floor cleaning machine may further comprise a solution tank to hold a cleaning liquid. In some embodiments, the solution tank is carried by the mobile body and has a capacity ranging from about 5L to about 15L.

In some embodiments, the floor cleaning machine may further comprise a recovery tank to hold a waste solution collected from the surface being cleaned. In some embodiments, the recovery tank is carried by the mobile body and has a capacity ranging from about 5L to about 15L.

In some embodiments, the floor cleaning machine may further comprise a hopper to hold the foreign materials collected from the surface. In some embodiments, the hopper is carried by the mobile body and has a capacity of up to about 5L.

In some embodiments, the one or more cleaning devices may comprise a brush, a squeegee, or a mop. In some embodiments, the one or more cleaning devices are releasably attachable to and detachable from the mobile body. In some embodiments, the one or more cleaning devices are magnetically attachable to the mobile body.

In some embodiments, the floor cleaning machine may further comprise an ultraviolet (UV) light sensor for sanitizing or disinfecting a waste water tank of the floor cleaning machine. In some embodiments, the floor cleaning machine may further comprise a bumper for detecting contact with one or more objects. In some embodiments, the floor cleaning machine may further comprise a processing unit configured to adjust a movement of the floor cleaning machine based on the detected contact in order to avoid or move around the one or more objects.

In some embodiments, the floor cleaning machine may further comprise a pressure adjustment system for the one or more cleaning devices. In some embodiments, the pressure adjustment system is configured to adjust a pressure applied to the surface by the one or more cleaning devices by adjusting a position or an orientation of the one or more cleaning devices. In some embodiments, the pressure adjustment system is configured to adjust a pressure applied to the surface based on a sensor output. In some embodiments, the sensor output is indicative of an amount of dirt, debris, or foreign materials on the surface.

In some embodiments, the floor cleaning machine comprises a floor scrubber. In some embodiments, the floor cleaning machine comprises an autonomous or semi-autonomous mobile robot. In some embodiments, the floor cleaning machine comprises one or more vision sensors and/or one or more navigation sensors. In some embodiments, the floor cleaning machine comprises a solution tank for storing water and/or a cleaning solution. In some embodiments, the floor cleaning machine is configured to receive a premeasured or predetermined dosage of a cleaning compound and to clean the surface using at least a portion of the premeasured or predetermined dosage of the cleaning compound. In some embodiments, an operational time or duration for the floor cleaning machine is based on a mode of operation or use for the floor cleaning machine. In some embodiments, the floor cleaning machine provides an optimal balance of size, weight, operating performance, and run time for cleaning an environment spanning up to about 15,000 square feet or more.

In some embodiments, the floor cleaning machine has a minimum turning radius while cleaning or performing a cleaning operation. In some embodiments, the minimum turning radius ranges from about 500 millimeters to about 800 millimeters. In some embodiments, the floor cleaning machine is configured to turn in place when the floor cleaning machine is not actively engaged in cleaning or performing a cleaning operation.

Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
**FIG. 1** schematically illustrates a robot, in accordance with some embodiments.
**FIG. 2** schematically illustrates an environment in which a robot may operate, in accordance with some embodiments.
**FIG. 3** schematically illustrates an environment comprising obstacles in which a robot may operate, in accordance with some embodiments.
**FIG. 4** schematically illustrates a perspective view of a robot, in accordance with some embodiments.
**FIG. 5** schematically illustrates a front view of a robot, in accordance with some embodiments.
**FIG. 6** schematically illustrates another front view of an exemplary robot, in accordance with some embodiments.
**FIG. 7** schematically illustrates a gap or opening in a chassis of a robot, in accordance with some embodiments.
**FIG. 8** schematically illustrates a side view of a robot, in accordance with some embodiments.
**FIG. 9** schematically illustrates an additional side view of an exemplary robot, in accordance with some embodiments.
**FIG. 10** schematically illustrates a bottom view of a robot, in accordance with some embodiments.
**FIG. 11** schematically illustrates an additional bottom view of an exemplary robot, in accordance with some embodiments.
**FIG. 12A** schematically illustrates an exemplary brush subsystem, in accordance with some embodiments. **FIG. 12B** and **FIG. 12C** schematically illustrate an exemplary side brush, in accordance with some other embodiments. **FIG. 12D** schematically illustrates an exemplary brush subsystem operating in combination with a squeegee, in accordance with some embodiments.
**FIG. 13** schematically illustrates a hopper, in accordance with some embodiments.
**FIG. 14** schematically illustrates a water tank, in accordance with some embodiments.
**FIG. 15** schematically illustrates various views of an exemplary water tank, in accordance with some embodiments.
**FIG. 16** schematically illustrates a squeegee that is attachable to a robot, in accordance with some embodiments.
**FIG. 17** schematically illustrates a squeegee that is attachable to a squeegee holder, in accordance with some embodiments.
**FIG. 18** schematically illustrates various view of an exemplary squeegee , in accordance with some embodiments.
**FIG. 19** schematically illustrates a top view of a robot, in accordance with some embodiments.
**FIG. 20** schematically illustrates a back view of a robot, in accordance with some embodiments.
**FIG. 21** schematically illustrates various components of an exemplary robot, in accordance with some embodiments.
**FIG. 22** schematically illustrates an exploded diagram of a robot comprising a water tank, in accordance with some embodiments.
**FIG. 23** schematically illustrates a plurality of robots and/or machines in communication with a central server, in accordance with some embodiments.
**FIGs. 24****,** **25****,** and **26** schematically illustrate a robot configured to move or maneuver under a structure, in accordance with some embodiments.
**FIG. 27** schematically illustrates an example of a brush assembly, in accordance with some embodiments.
**FIG. 28** schematically illustrates a computer system that is programmed or otherwise configured to implement any of the methods provided herein.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

The term "real time" or "real-time," as used interchangeably herein, generally refers to an event (e.g., an operation, a process, a method, a technique, a computation, a calculation, an analysis, a visualization, an optimization, etc.) that is performed using recently obtained (e.g., collected or received) data. In some cases, a real time event may be performed almost immediately or within a short enough time span, such as within at least 0.0001 millisecond (ms), 0.0005 ms, 0.001 ms, 0.005 ms, 0.01 ms, 0.05 ms, 0.1 ms, 0.5 ms, 1 ms, 5 ms, 0.01 seconds, 0.05 seconds, 0.1 seconds, 0.5 seconds, 1 second, or more. In some cases, a real time event may be performed almost immediately or within a short enough time span, such as within at most 1 second, 0.5 seconds, 0.1 seconds, 0.05 seconds, 0.01 seconds, 5 ms, 1 ms, 0.5 ms, 0.1 ms, 0.05 ms, 0.01 ms, 0.005 ms, 0.001 ms, 0.0005 ms, 0.0001 ms, or less.

### Overview

The present disclosure provides robotic systems that are compact in size, highly maneuverable, efficient and productive, and powerful enough for commercial cleaning operations in environments spanning up to 15,000 square feet or more. The form factors of the presently disclosed robotic systems allow for precise cleaning in and around tight areas while avoiding collisions with obstacles, and the selection and configuration of components allows the robotic systems to clean large areas or volumes efficiently. The combination of form factor and cleaning performance as exemplified in the various embodiments described herein enables both meticulous cleaning of hard to reach spots and high productivity rates / area coverage. The robotic systems disclosed herein provide an optimal balance between size and cleaning performance, and can be configured to clean many different types of environments thoroughly and effectively.

### Robot / Machine

In an aspect, the present disclosure provides a system comprising a robot or a machine. In some embodiments, a machine may comprise an autonomous, semi-autonomous, and/or non-autonomous robot or machine. In some embodiments, a robot may comprise an autonomous, semi-autonomous, and/or non-autonomous machine or robot. In some embodiments, a robot may be referred to interchangeably as a machine, and a machine may be referred to interchangeably as a robot. In some cases, a robot may be equivalent to a machine, and vice versa. Alternatively, a robot may comprise a system that is capable of operating autonomously or semi-autonomously, and a machine may comprise a non-autonomous system that is capable of being operated by a human or another machine or robot.

In some embodiments, the robots or machines may comprise, for example, a non-autonomous, semi-autonomous, or autonomous vehicle, a rover, a drone, or a shuttle for transporting humans or objects. In some cases, the robots or machines may comprise a humanoid robot or a non-humanoid robot. In some cases, the robots or machines may comprise a cleaning machine or robot (e.g., a floor scrubber or a vacuum).

In any of the embodiments described herein, the one or more robots or machines may be configured to operate individually or collectively as a fleet or a swarm of robots or machines. The term "fleet" as used herein may refer to any grouping or collection of a plurality of robots or other machines that are independently or jointly controllable by a human or a computer system. The fleet may comprise one or more robots and/or one or more machines. The one or more robots and/or the one or more machines may comprise a non-autonomous, semi-autonomous, or autonomous robot or machine that can be controlled either locally or remotely. The robots and/or machines in the fleet may be controlled by a human operator and/or a computer. In any of the embodiments described herein, the fleet may comprise a combination of robots and/or machines. In any of the embodiments described herein, the fleet may comprise a combination of autonomous, semi-autonomous, and/or non-autonomous robots and/or machines.

In some embodiments, the robots or machines may comprise a non-autonomous robot or machine. Such non-autonomous robot or machine may not or need not comprise or have autonomous navigation functions or capabilities. In some cases, such non-autonomous robot or machine may be configured to operate based on one or more inputs, commands, or instructions provided by a human operator. The one or more inputs, commands, or instructions may comprise a physical motion to move the robot or machine, an auditory communication, or a virtual input or selection of an action or movement to be performed by the robot or machine.

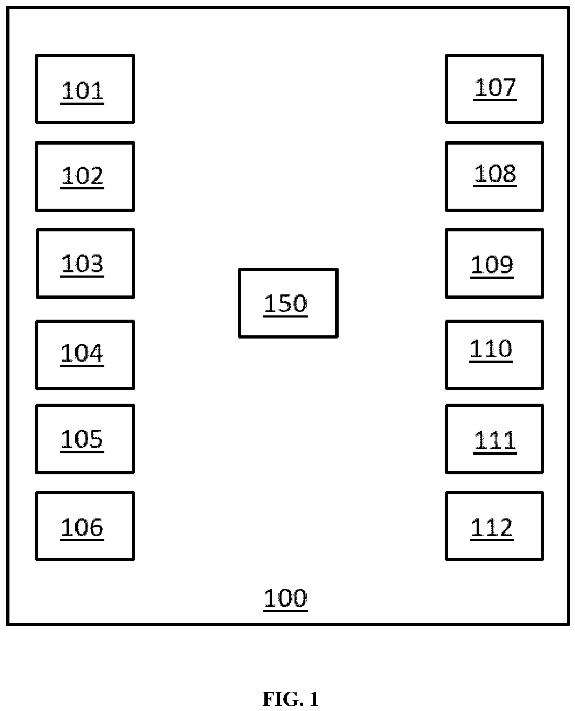

**FIG. 1** illustrates an example of a robot 100. The robot 100 may be configured to execute a cleaning routine or a cleaning operation. The cleaning routine or a cleaning operation may involve using an instrument, a tool, or a substance (e.g., water and/or detergent) to clean, sanitize, or disinfect an area or a region.

In some embodiments, the robot 100 may comprise a drive unit 101. The drive unit 101 may comprise, for example, wheels, rollers, conveyor belts, treads, magnets, and the like.

In some embodiments, the robot 100 may comprise one or more brushes 102. The brushes 102 may be operated to clean an environment. The brushes 102 may be rotatable to capture dirt, dust, debris, or waste materials or particles. In some cases, the brushes 102 may comprise a scrubber.

In some embodiments, the robot 100 may comprise a hopper 103. The hopper 103 may be configured to collect garbage from a brush or scrubber that is proximal to the hopper 103.

In some embodiments, the robot 100 may comprise a tank 104. The tank 104 may comprise a solution tank and a waste water tank. The solution tank may contain either (a) cleaning solution (e.g. clean water with detergent added) or (b) clean water. In some cases, the cleaning machine may be configured to automatically mix detergent and clean water to produce a cleaning solution that can be applied to the floor. In some cases, the solution tank can be manually filled by a user with a pre-mixed cleaning solution. In other cases, the solution tank can be manually filled by a user with clean water and detergent separately, which can then be mixed to produce a cleaning solution. In some embodiments, the solution in the solution tank may be sprayed onto the rolling brush and/or the side brushes for cleaning the ground. In some embodiments, a negative pressure can be applied to collect the waste water on the ground back to the waste water tank.

In some embodiments, the robot 100 may comprise a handle 105. The handle 105 may be used to operate, push or carry the robot. In some cases, the handle can be used to engage an operational mode, to control the robot in a selected mode, or to switch between different operational modes.

In some embodiments, the robot 100 may comprise a squeegee 106. The squeegee 106 can be used to clean or remove water residue or water marks from the area being cleaned.

In some embodiments, the robot 100 may comprise a bumper 107. The bumper 107 can be configured to detect a contact (e.g., an impact or a collision) between the robot 100 and one or more objects or personnel or obstacles in a cleaning environment. The bumper 107 can be used to protect the robot 100 or any components of the robot 100 from being damaged.

In some embodiments, the robot 100 may comprise a cleaning detergent distribution subsystem 108. The cleaning detergent distribution subsystem may be configured to provide or release detergent into a water tank 104 of the robot 100. In some cases, the detergent may be provided in a pre-measured dosage within one or more consumable packs, containers, or pods.

In some embodiments, the robot 100 may comprise a treatment or sanitization subsystem 109. The treatment or sanitization subsystem 109 may be configured to perform a treatment operation (e.g., a sanitization operation or a disinfection operation) for one or more components or portions of the robot 100. In some cases, the treatment or sanitization subsystem may be used to treat or sanitize a hazardous or toxic material or any other material that can be harmful to human or animal health.

In some embodiments, the robot 100 may comprise a navigation subsystem 110. The navigation subsystem 110 may be configured to provide or manage a control logic used by the robot 100 to navigate an environment or to execute a cleaning operation or procedure.

In some embodiments, the robot 100 may comprise a communications unit 111. The communications unit 111 may comprise a transmitted and/or a receiver for transmitting and/or receiving information or data. The information or data may comprise operational data for the robot, including data of the components of the robot. In some cases, the communications unit 111 may be configured to transmit the information or data to a central server or one or more other robots or machines. In some cases, the communications unit 111 may be configured to receive information or data transmitted to the robot 100 from a central server or one or more other robots or machines.

In some embodiments, the robot 100 may comprise one or more sensors 112. The one or more sensors 112 may be used to obtain measurements associated with an operation of the robot (or any components or subsystem thereof), the environment in which the robots operates, or the obstacles around the robot. In some cases, the robot may use the measurements obtained using the one or more sensors 112 to control or adjust robot operation (e.g., navigation of the robot through an environment, or operation of one or more components or subsystems of the robot).

In some embodiments, the robot 100 may comprise a processor 150. The processor 150 may be configured to control an operation of the robot (or any components or subsystem thereof) based on the measurements or readings obtained using the one or more sensors 112. In some cases, the processor 150 may be operatively coupled to the sensors 112 and/or various other components or subsystems of the robot 100 to aid in (1) processing of sensor data and (2) controlling an operation or a behavior of the robot 100 or various components/subsystems of the robot 100.

### Environment

In some embodiments, the robot may be optimized or configured for floor cleaning for commercial use. The robot can service such areas much more effectively and efficiently than (i) large commercial cleaning robots that cannot maneuver tight spaces nimbly or precisely navigate to or through hard to reach areas, (ii) small household cleaning robots that lack the battery capacity or productivity rate of robots for commercial cleaning applications, and (iii) human operators manually cleaning the areas using mops or buckets.

In some embodiments, the systems and methods of the present disclosure may be used to clean an environment. The environment may comprise an indoor environment or an outdoor environment. In some cases, the environment may comprise a combination of one or more indoor environments and one or more outdoor environments. The indoor environment may comprise, for example, a building, an office, a home, a store, or any other space or area that is at least partially enclosed by one or more walls, ceilings, panels, flooring, or other structural elements. The outdoor environment may comprise, for example, any space that is at least partially exposed to the natural elements, including, for example, public spaces, private spaces that are not enclosed by a structural element or component, roadways, terrestrial or aquatic ecosystems, and the like. In some embodiments, the robot may clean or may be configured to clean places such as a retail store, a fast food restaurant, a convenience store, an airport, a railway station, a shopping mall, a commercial building, a super market, a campus, or a school.

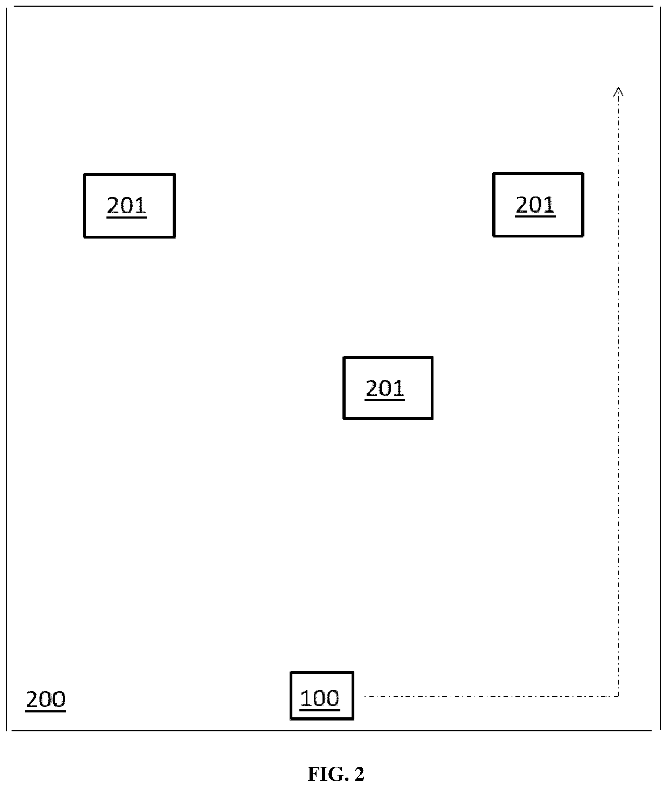

**FIG. 2** illustrates an exemplary environment 200 in which the robot 100 may operate. The environment may comprise one or more obstacles 201 for the robot 100 to navigate around. In any of the embodiments described herein, the robot 100 may be configured to clean the environment 200 while navigating around the one or more obstacles 201.

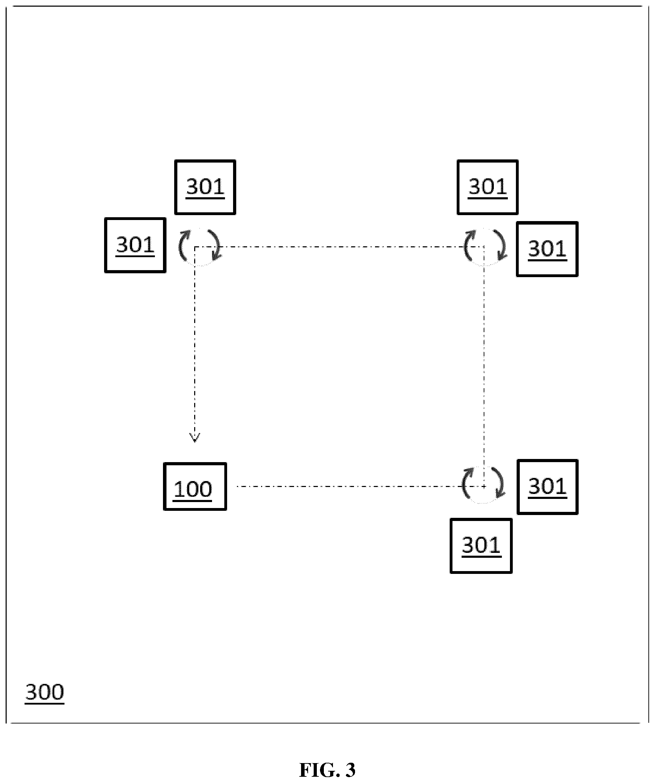

As shown in **FIG. 3****,** the robot 100 may be capable of navigating environments 300 in which a plurality of obstacles 301 are closely positioned or clustered next to each other. The plurality of obstacles 301 may comprise separate and distinct obstacles. Alternatively, the plurality of obstacles 301 may comprise different portions, regions, section, or components, of a same object (e.g., different legs of a same chair or table). In any of the embodiments described herein, the robot 100 may comprise a form factor that allows the robot 100 to navigate around the plurality of obstacles 301, make tight turns with a minimal turning radius, and maintain a precise cleaning path despite the presence of obstacles along or near the robot's trajectory of motion. The cleaning path may be dynamically adjusted by the robot to account for the particular characteristics or layout of the cleaning environment while taking advantage of the robot's form factor and ability to make tight turns with a minimal turning radius.

### Benefits / Advantages

As described elsewhere herein, the robot can be compact in size, highly maneuverable, efficient and productive, and powerful enough for commercial cleaning operations in environments spanning up to 15,000 square feet or more. The form factor of the robot can allow for precise cleaning in and around tight areas while avoiding collisions with obstacles. The selection and configuration of components can allow the robot to clean large areas or volumes efficiently. The combination of form factor and cleaning performance may enable both meticulous cleaning of hard to reach spots and high productivity rates / area coverage. The robot can provide an optimal balance between size and cleaning performance (including, for example, tank size, motor size, and battery size), and can be configured to clean many different types of environments thoroughly and effectively.

In addition to the benefits above, the robot has many other advantageous features, such as an optimal balance of size, weight, operating performance, and run time. The robot may have a form factor that allows it to move nimbly around a cleaning environment, without tipping over when traversing uneven grounds. The robot may have a square-shaped base, profile, or form factor that allows it to navigate crowded spaces and turn in place. In some cases, the robot may be configured to rotate or turn in place as needed (e.g., to reorient the robot in a desired direction without requiring excessive movement that could cause the robot to collide with an object in close proximity to the robot). The robot may also have a minimal turning radius to give the squeegee of the robot enough space to operate and pick up any remaining water on the floor. In some embodiments, the positioning or placement of the robot's mechanical parts close to the ground can help to maximize the scrubbing effect applied by the brushes of the robot. The robot may have a reduced weight and volume compared to other cleaning robots in the space while providing better cleaning performance, higher productivity, and longer operating times.

### Robot / Machine Form Factor

In some embodiments, the robot may have a range of specifications. Exemplary ranges of the robot specifications are shown below in **Table A1.**

**Table A1. Specification of machines, in accordance with some embodiments.**

| | **Exemplary Specification** | **Exemplary Ranges** |
|---|---|---|
| Dimensions (Length) | 480 mm | 350-650 mm |
| Dimensions (Width) | 500 mm | 300-550 mm |
| Dimensions (Height) | 700 mm | 500-800 mm |
| Voltage | 24 V | 12-36 V |
| Battery Capacity | 25 Ah (600WH) | 200-900 WH |
| Running Time | Between 1.5 and 3 hours | 0.5-4 hours |
| Recovery Tank Capacity | 11 L | 5-20 L |
| Solution Tank Capacity | 10L | 5-20 L |
| Hopper Capacity | 0.4 L | 0-5 L |
| Cleaning Path | 480 mm | 250-510 mm |
| Productivity Rate Projected | 500 m²/h | 125-1800 m²/h |
| Maximum Travel Speed (Autonomous) | 0-1.8 km/h | 0-3.6 km/h |
| Side Brush Size | 2 x 0100mm | |
| Brush Size | 70 mm x 330 mm (Cylindrical) | |
| Flow Rate | 150 mL/min (Maximum), 130 mL/min (Eco-friendly mode) | 0-300 mL/min |
| Minimum Turn-Around Aisle Width (Autonomous) | 700 mm | 500-800 mm |
| Weight (excluding battery and assuming tanks are empty) | 55 kg (65 kg with water) | 30-80 kg |

In some cases, it may be advantageous for the robot to comprise a small footprint. In some cases, a small footprint may allow easy storage of the robot (e.g., in a relatively small storage area), easy transport of the robot (e.g., transport by hand or a cart), and/or easy accessibility of the robot in small areas for cleaning (e.g., under a table).

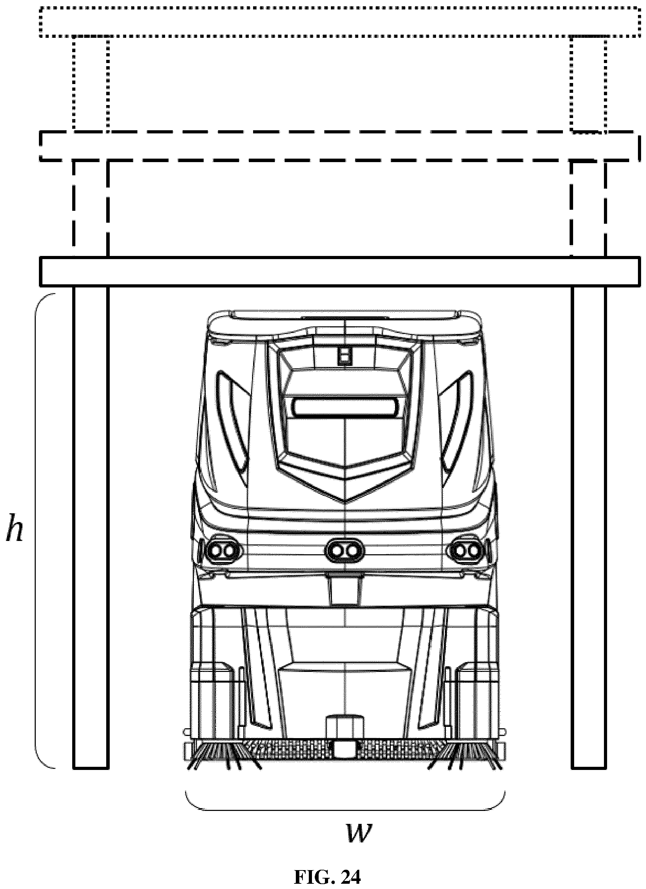

**FIG. 24** schematically illustrates a robot having dimensions that allow the robot to maneuver under various structures (e.g., a table or a desk) with sufficient clearance, unlike other commercial cleaning machines that are too large to fit under such structures. In some embodiments, the robot may have a height and/or width that allows the robot to traverse under furnishing or other structures present in a cleaning environment. In some embodiments, the height of the robot may be adjustable (e.g., to fit under certain furnishings or structures). For example, the robot may lower its height before going underneath a table or a desk.

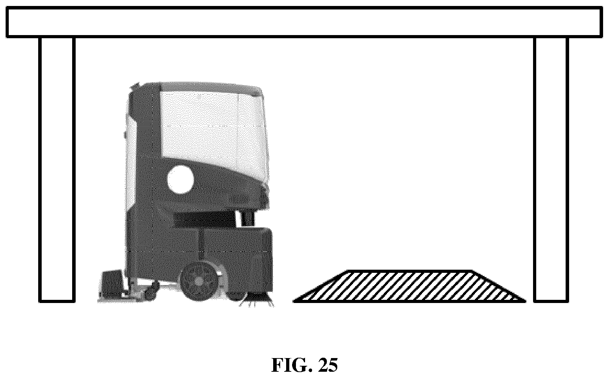

In some embodiments, the robot may be configured to adjust its ground clearance. For example, **FIG. 25** shows a robot that is traversing under a structure, in accordance with some embodiments. In some cases, the robot may be configured to adjust its height and/or ground clearance if there are obstacles (bumps or raised features) on a surface being traversed or to be traversed by the robot. This can prevent the machine from toppling when traversing those features, or otherwise becoming immobilized or stuck due to the raised features.

In any of the embodiments described herein, the robot may be configured to adjust its height and/or ground clearance. In some cases, the robot may be configured to raise or lower its height. In some cases, the robot may be configured to raise or lower its ground clearance. The robot may adjust its height and/or ground clearance based on the sensing of the environment or the obstacles in the environment. The robot may adjust its height and/or ground clearance based on the robot's navigation path or cleaning routine.

### Weight

In some embodiments, the robot may comprise a weight of about 55 kg. In some embodiments, the robot may comprise a weight of about 65 kg. In some embodiments, the robot may comprise a weight of at least about 15, 20, 25, 30 35, 40, 45, 50, 55, 60, 65, 70, 75, 80, 85, 90, 95, or 100 kg. In some embodiments, the robot may comprise a weight of at most about 15, 20, 25, 30 35, 40, 45, 50, 55, 60, 65, 70, 75, 80, 85, 90, 95, or 100 kg. In some embodiments, the weight may be measured when the robot comprises a full tank. In some embodiments, the weight may be measured when the robot comprises an empty tank.

### Volume / Size

In some embodiments, the robot may comprise a length of about 480 mm. In some embodiments, the robot may comprise a length of at least about 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, or 700 mm. In some embodiments, the robot may comprise a length of at most about 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, or 700 mm.

In some embodiments, the robot may comprise a width of about 500 mm. In some embodiments, the robot may comprise a width of at least about 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, or 600 mm. In some embodiments, the robot may comprise a width of at most about 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, or 600 mm.

In some embodiments, the robot may comprise a height of about 700 mm. In some embodiments, the robot may comprise a height of at least about 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, or 800 mm. In some embodiments, the robot may comprise a height of at most about 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, or 800 mm.

In some embodiments, the robot may comprise a volume of about 0.168 m³. In some embodiments, the robot may comprise a volume of at least about 0.01, 0.02, 0.03, 0.04, 0.05, 0.06, 0.07, 0.08, 0.09, 0.1, 0.2, or 0.3 m³. In some embodiments, the robot may comprise a volume of at most about 0.01, 0.02, 0.03, 0.04, 0.05, 0.06, 0.07, 0.08, 0.09, 0.1, 0.2, or 0.3 m³.

### Traveling speed

In some embodiments, the robot may be operable to travel at a speed of about 1.8 km/h. In some embodiments, the robot may be operable to travel at a speed of at least about 0, 0.6, 1.2, 1.8, 2.4, 3, or 3.6 km/h. In some embodiments, the robot may be operable to travel at a speed of at most about 0.6, 1.2, 1.8, 2.4, 3, or 3.6 km/h.

### Turning Radius

In any of the embodiments described herein, the robot may be configured to turn in place (e.g., when the robot is moving through or navigating an area, but not actively cleaning). In some embodiments, the robot may be configured to turn with a minimum turning radius (e.g., during a cleaning operation) to ensure that the squeegee can perform its job of wiping off any excess solution and/or water from the floor. In some embodiments, the robot may have a minimum turning radius of about 500 mm during a cleaning operation. In some embodiments, the robot may have a minimum turning radius of at least about 500, 600, 700, or 800 mm during a cleaning operation. In some embodiments, the robot may have a minimum turning radius of at most about 500, 600, 700, or 800 mm during a cleaning operation. In some cases, the robot's minimum turning radius may change depending on whether or not the robot is operating in a cleaning mode (e.g., whether or not the robot is performing a cleaning operation). In some cases, the robot may be configured to turn in place (i.e., its minimum turning radius may be zero). In some embodiments, the robot's ability to turn or rotate in place may be enabled by its square shape profile, which may be advantageous over other robot form factors that are based on a rectangular or round shape profile. In some cases, the square shape profile may allow the robot to clean corners while still maintaining the ability to turn in place and overall robot maneuverability during a cleaning operation (e.g., by way of a tight turning radius during cleaning).

### Battery

In some embodiments, the robot may comprise a battery. In some embodiments, the battery may comprise an operational voltage that is safe for a human being. In some embodiments, the battery may comprise an operating voltage of about 24 V. In some embodiments, the battery may comprise an operating voltage of at least about 12, 18, 24, 30, or 36 V. In some embodiments, the battery may comprise an operating voltage of at most about 12, 18, 24, 30, or 36 V. In some embodiments, the battery may comprise a capacity of about 600 Wh. In some embodiments, the battery may comprise a capacity of at least about 200, 300, 400, 500, 600, 700, 800, 900, 1000, 1100, or 1200 Wh. In some embodiments, the battery may comprise a capacity of at most about 200, 300, 400, 500, 600, 700, 800, 900, 1000, 1100, or 1200 Wh. In some embodiments, the battery may be rechargeable.

### Performance

In some embodiments, the robot may be capable of an operation time of about 1 hour on a full battery. In some embodiments, the robot may have an operation time of at least about 0.5, 1, 1.5, 2, 2.5, 3, 3.5, or 4 hours. In some embodiments, the robot may have an operation time of at most about 0.5, 1, 1.5, 2, 2.5, 3, 3.5, or 4 hours. In some embodiments, the robot may be configured to operate on battery power for up to 4 hours or more. In some embodiments, the robot may operate for 1.5 to 2 hours in maximum performance mode, 3.5 to 4 hours in eco mode, and up to 15.5 hours or more in a mop only mode (whereby the brush motor and vacuum motors are turned off).

In some embodiments, the robot may have a productivity rate of about 500 m²/h. In some embodiments, the robot may have a productivity rate of at least about 100, 200, 300, 400, 500, 600, 700, 800, 900, 1000, 1100, 1200, 1300, 1400, 1500, 1600, 1700, or 1800 m²/h. In some embodiments, the robot may have a productivity rate of at most about 100, 200, 300, 400, 500, 600, 700, 800, 900, 1000, 1100, 1200, 1300, 1400, 1500, 1600, 1700, or 1800 m²/h. The productivity rate may correspond to an area cleaned per unit time.

### Components and Subsystems

In some aspects, the present disclosure provides a compact robot for cleaning. The compact robot may have any of the dimensions, specifications, or operational characteristics described herein.

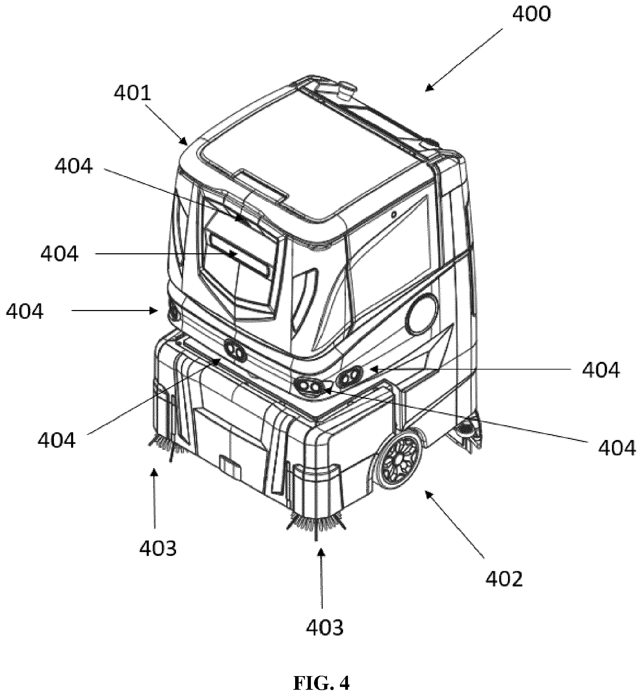

**FIG. 4** illustrates an example configuration for a robot 400. The robot 400 may comprise a chassis 401 and a drive mechanism 402 for moving the robot 400 through a cleaning environment. In some embodiments, the robot may comprise one or more brushes 403 for cleaning one or more objects, surfaces, or regions in the cleaning environment. In some embodiments, the robot may comprise one or more sensors 404 for detecting objects in the cleaning environment or mapping/visually inspecting the cleaning environment. The one or more sensors 404 may be positioned anywhere on the robot in any suitable configuration to allow the robot to intelligently sense obstacles or movement around the robot from multiple directions or perspectives. The placement of the sensors 404 may provide up to a 360 degree coverage for the robot to detect or sense obstacles or movement (e.g., peripheral movement).

### Chassis

In some embodiments, the robot comprises a chassis. The chassis may comprise a housing for various components or subsystems of the robot. In some cases, the chassis may be reinforced to prevent the robot from tipping over when travelling on an uneven surface.

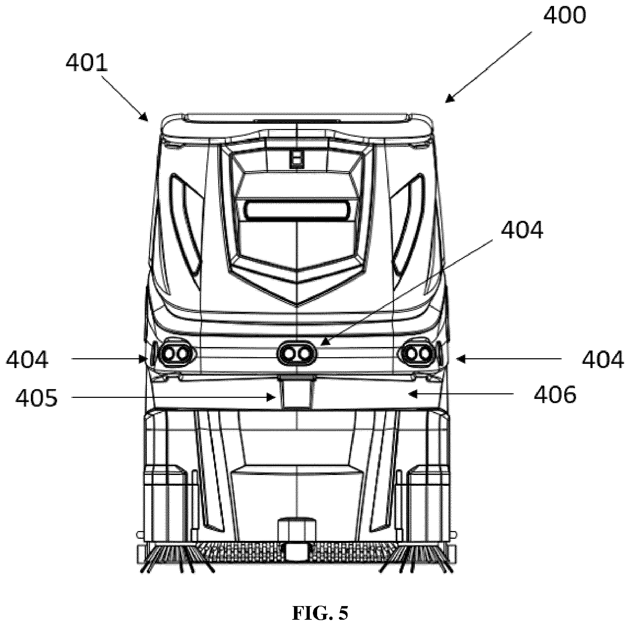

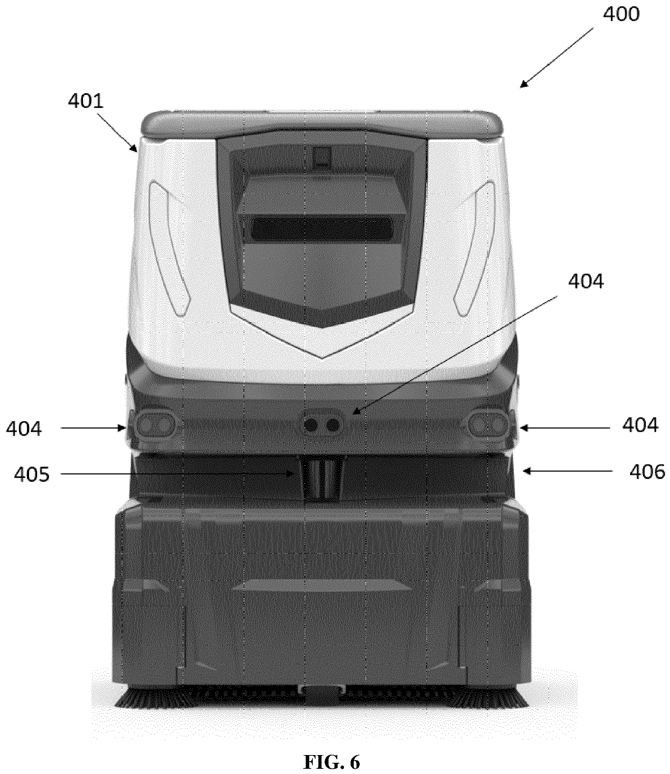

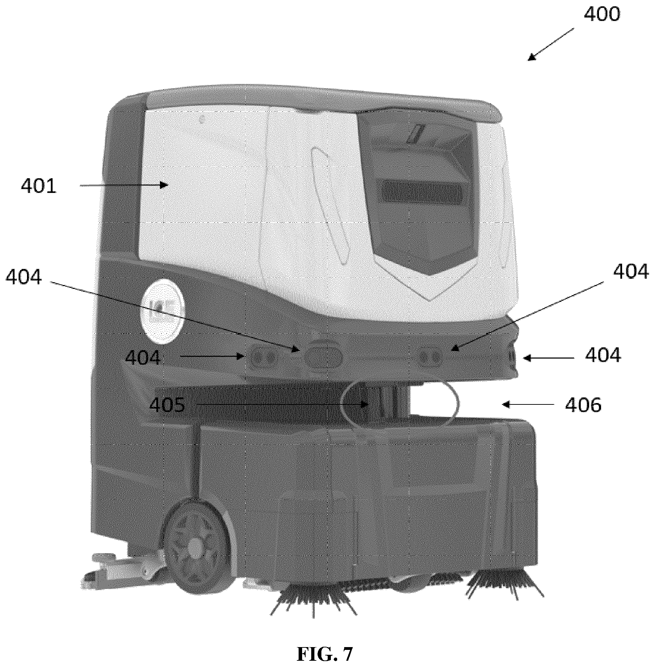

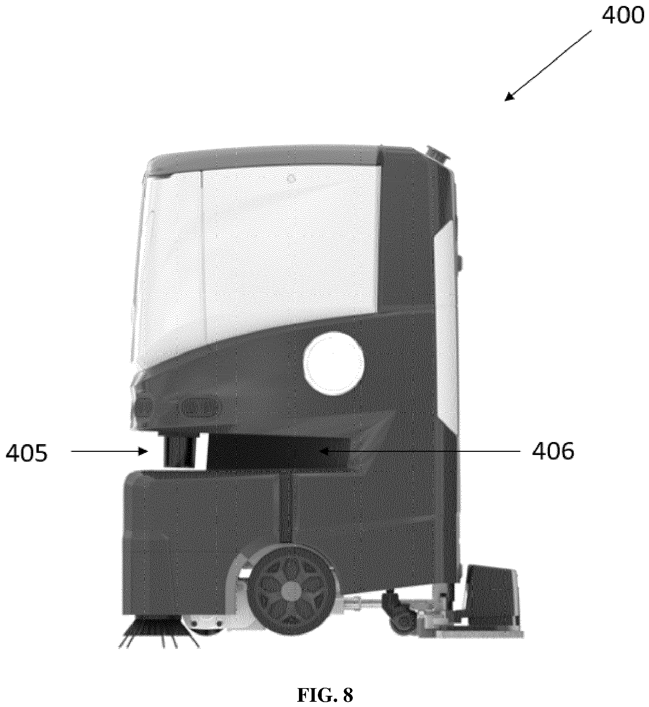

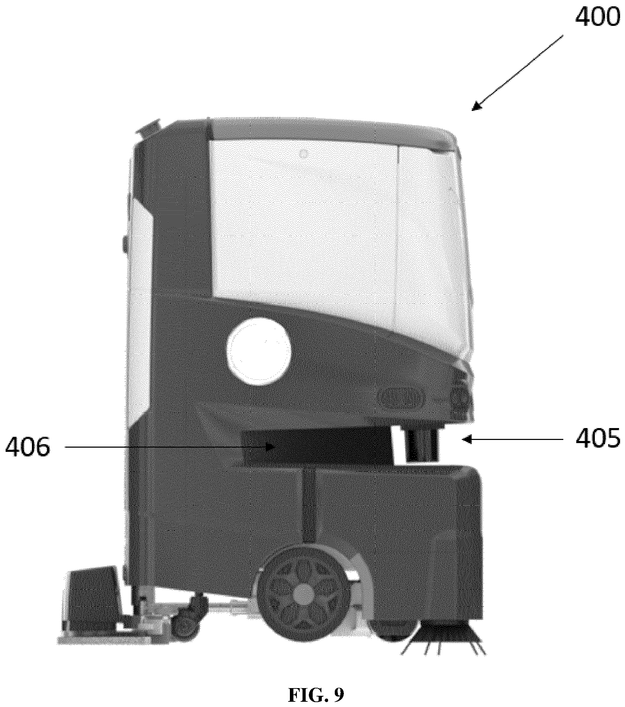

Referring to **FIG. 5****,** **FIG. 6****,** and **FIG. 7****,** in some cases the robot 400 may comprise a gap or opening 406 in the body or chassis 401 of the robot 400. In some embodiments, the robot 400 may comprise one or more sensors 405 (e.g., LIDAR sensors) positioned in the gap or opening 406 in the body or chassis 401 of the robot 400. **FIG. 8** and **FIG. 9** schematically illustrate side views of the robot 400 and the gap or opening 406 in the body or chassis of the robot 400. In some cases, a sensor 405 (e.g., an optical sensor, a LIDAR sensor, a radar sensor, etc.) may be integrated on a portion of the robot 400 that is provided within or proximal to the gap or opening 406. In some cases, a sensor 405 that is provided within or proximal to the gap or opening 406 may have a wide horizonal field-of-view. In some cases, the wide horizonal field-of-view may be at least about 180 degrees, 225 degrees, or 270 degrees. In some cases, a sensor 405 that is provided in a middle of the body or chassis of the robot 400 may be able to directly detect objects nearby the robot. In some cases, a sensor 400 may be positioned to have minimal blind spots for the robot.

### Drive Mechanism

In some embodiments, the robot may comprise a drive mechanism to transport the robot. The drive mechanism may comprise wheels, conveyor belts, treads, rollers, magnets, and the like. In some cases, the drive mechanism may not or need not comprise a transaxle. The drive mechanism may allow the robot to turn in place 360 degrees on the ground (thereby minimizing the robot turning radius) and maneuver in tight spots or areas.

### Brushes

In some embodiments, the robot may comprise one or more brushes. The one or more brushes may comprise a brush assembly. In some embodiments, the brush assembly comprises a cylindrical rolling brush. The cylindrical rolling brush may be configured to process or displace debris or other foreign objects that are in front of the robot. In some embodiments, the brush assembly comprises two disc-shaped side brushes. The cylindrical rolling brush may be positioned substantially between the two disc-shaped side brushes. In some embodiments, the brush assembly may further comprise a trash hopper behind the cylindrical rolling brush. In some embodiments, the brush assembly may further comprise a drive wheel assembly. In some embodiments, the brush assembly may further comprise a squeegee assembly.

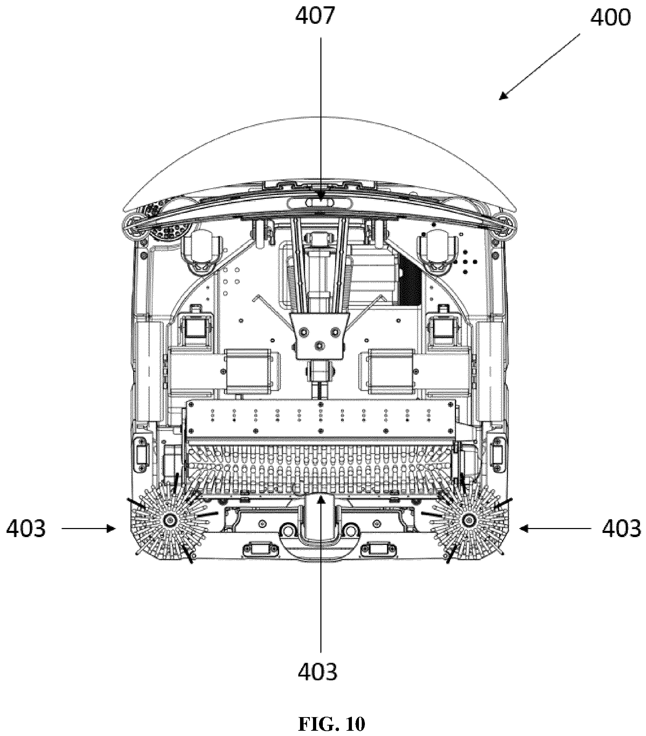

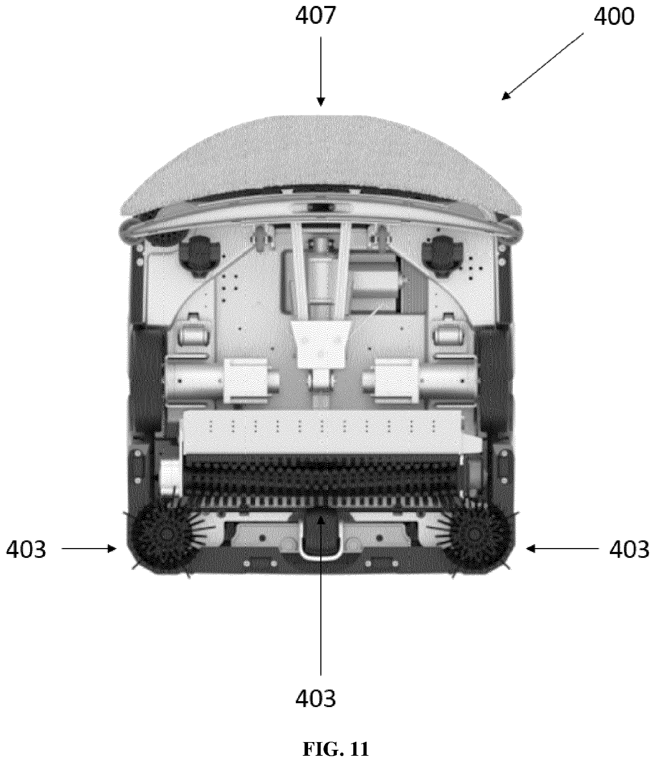

In some embodiments, the robot may comprise at least one main brush and at least two side brushes. The at least one main brush and at least two side brushes may be configured for different cleaning tasks or purposes. **FIG. 10** and **FIG. 11** illustrate a bottom view of an exemplary robot 400 comprising a plurality of brushes 403. The plurality of brushes 403 may comprise the at least one main brush and the at least two side brushes. In some cases, the at least two side brushes may be positioned at a corner or an edge of the robot 400 to aid in the cleaning or brushing of corner regions in a cleaning environment. In some cases, the robot may have brushes positioned at a corner or edge of the robot. This can aid in cleaning corners or other tight spaces. In any of the embodiments described herein, the robot may have a weight distribution which allows the robot to effectively utilize the various brushes at sufficient pressure to maintain optimal cleaning results even when operating on uneven surfaces.

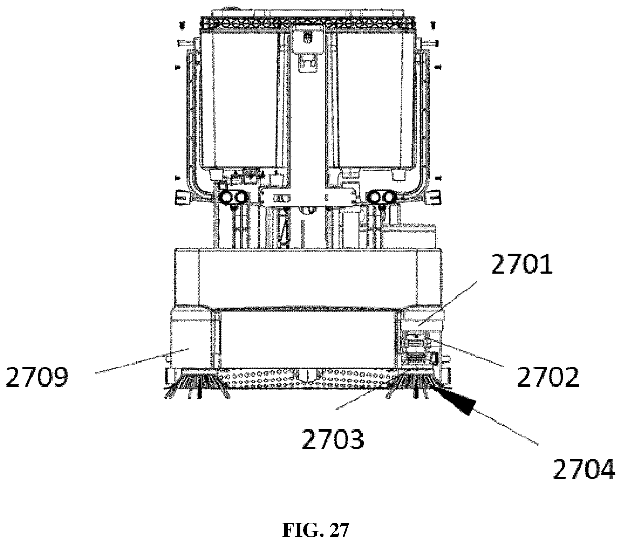

In some embodiments, a robot may comprise a brush. In some embodiments, the brush may be used for floor sweeping, floor scrubbing, floor washing, or any combination thereof. In some cases, it may be advantageous to have a brush that has minimized dead corners (e.g., places where a brush cannot reach or has difficulty in reaching for cleaning and/or applying sufficient pressure for cleaning). **FIG. 27** schematically illustrates a brush assembly, in accordance with some embodiments. In some cases, it may be advantageous to have a brush that is configured to avoid or minimize collision with obstacles while cleaning, because collision may damage the brush. In some embodiments, a brush may comprise a mounting portion 2701, an adjusting portion 2702, a base portion 2703, a brush body 2704, or any combination thereof. In some embodiments, the base portion 2703 can be rotatably coupled in the mounting portion 2701. In some embodiments, the adjusting portion 2702 can be provided between the mounting portion 2701 and the base portion 2703. In some embodiments, the adjusting portion 2702 may be provided with springs.

In some embodiments, a body of a brush 2704 may comprise long bristles, short bristles, outer bristles, or any combination thereof. In some embodiments, the long bristles and/or the short bristles may be provided at a lower side of the base portion 2703. In some embodiments, the outer bristles may be provided at an external side of the base portion 2703. In some embodiments, the long bristles, short bristles, outer bristles, or a combination thereof may be efficient at cleaning dead corners. In some embodiments, springs may be provided with the brush. In some embodiments, the springs may reduce or prevent damage to the brush in a cleaning operation.

In some embodiments, the long bristles and the short bristles may be arranged in an alternating order, e.g., "long-short-long-short." In some embodiments, the long bristles, the short bristles, and/or the outer bristles may be orientated obliquely.

In some embodiments, a protective housing 2709 may be provided at an exterior of the mounting portion 2701 and a base portion 2703. In some embodiments, the protective housing may reduce or prevent collision damage to the brush. In some embodiments, the protective housing 2709 may comprise an elastic material.

In some embodiments, a rotary shaft may be provided with the brush. In some embodiments, the rotary shaft may pass through a center of an adjusting portion 2702. In some embodiments, the rotary shaft may extend from a mounting portion 2701. In some embodiments, the adjusting portion 2702 may include one or more, or a plurality of adjustment rods. In some embodiments, springs may be provided to the adjustment rod. In some embodiments, an upper end of the adjustment rod may abut against the mounting portion 2701. In some embodiments, a lower end of the adjustment rod may abut against the base portion 2703. In some embodiments, the adjustment rod may be configured to adjust a height of the brush body 2704.

In some embodiments, a ring-shape protection may be provided to the adjustment rod. In some embodiments, the ring-shape protection may comprise elastic material. In some embodiments, the ring-shape protection may reduce or prevent damage to the adjustment rod during a cleaning operation.

In some embodiments, the brush may comprise a floating brush mechanism. In some embodiments, a robot may comprise a left brush and a right brush. In some embodiments, the brush may be installed on a brush head assembly. In some embodiments, the left brush may be installed on a brush head assembly. In some embodiments, the right brush may be installed on a brush head assembly. In some embodiments, the brush may be installed on the brush head assembly by one or more guide posts (e.g., four guide posts). In some embodiments, the one or more guide posts may be disposed perpendicular to a horizontal plane (e.g., in reference to the ground). In some embodiments, the brush may float in a vertical direction within a predetermined stroke.

In some embodiments, a pressure may applied onto the ground in an operational state. In some embodiments, a pressure may applied onto the ground with springs installed on a guide post. In some embodiments, a pressure may applied onto the ground with springs installed on four guide posts. In some embodiments, the brush head assembly may be lifted up from the ground. In some embodiments, the brush head assembly may be lifted up from the ground in a non-operational state. In some embodiments, a side brush may be lifted up to a predetermined height. In some embodiments, a side brush may be lifted up by a set of pulley mechanism and/or a steel wire. In some embodiments, the set of pulley mechanisms and/or the steel wire may facilitate installation and/or detachment of the side brush.

In some embodiments, the left side brush and/or the right side brush may be each installed on a brush head assembly by 1, 2, 3, or 4 guide posts. In some embodiments, the guide posts may be disposed perpendicular to the horizontal plane (e.g., in reference to the ground). In some embodiments, the brush may be floating in a vertical direction within a predetermined stroke. In some embodiments, the guide post may be provided with a compressed spring. In some embodiments, the side brush may be coupled to a chassis. In some embodiments, the side brush may be coupled to a chassis via a steel wire and/or a set of pulley mechanisms.

In some embodiments, the brush head assembly is lowered to touch the ground in an operation state. In some embodiments, no force is applied on the steel wire in the operational state. In some embodiments, a pressure is applied onto the side brush from the spring in the operational state. In some embodiments, the brush head assembly is lifted up in a non-operational state. In some embodiments, the side brush is lifted up to a predetermined height by the set of pulley mechanism and/or the steel wire to facilitate installation and detachment of the side brush in the non-operational state.

In some embodiments, the robot may comprise one or more floating brushes that can be controlled to adjust a pressure applied by the brushes to a cleaning surface. The pressure can be adjusted to optimize brushing efficiency.

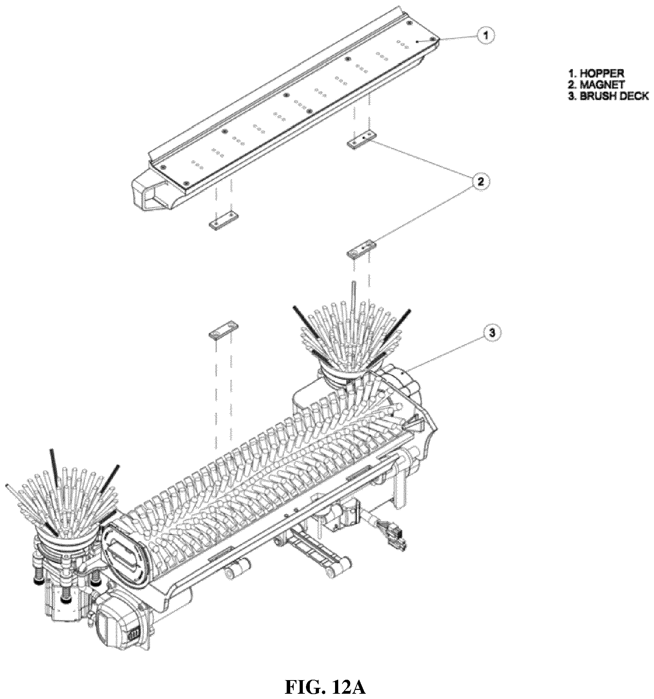

**FIG. 12A** illustrates an exemplary brush subsystem for the robot. The brush subsystem may comprise a plurality of rotatable brushes. The plurality of rotatable brushes may comprise a first brush configured to rotate along a first axis and a second brush configured to rotate along a second axis that is different than the first axis. In some cases, the plurality of rotatable brushes may comprise a first rotatable brush positioned between a second and third rotatable brush. The plurality of rotatable brushes may have different sizes, shapes, and/or functionalities. In some cases, the brush subsystem may comprise a brush deck that is magnetically attachable to a hopper as described elsewhere herein. The brushes may be configured to rotate clockwise or counter-clockwise relative to a surface on which the robot is positioned or moving.

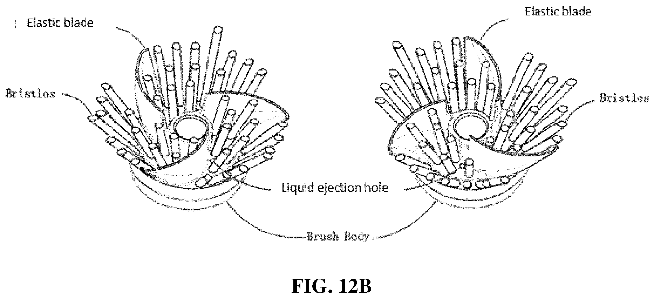

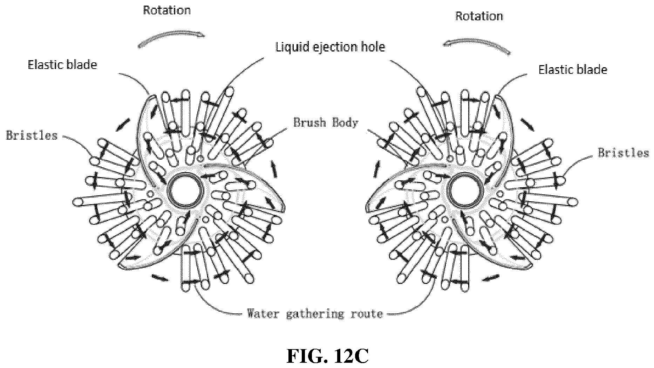

**FIG. 12B** illustrates a perspective view, and **FIG. 12C** illustrates a bottom view, of a side brush system in accordance with some embodiments. The side brush subsystem may comprise a plurality of side brushes. For example, the side brush subsystem may comprise 2, 3, 4 or more side brushes. Referring to **FIG. 12B****,** a side brush may comprise a base (side brush body) having one or more liquid ejection holes. Cleaning liquid from a solution tank of the robot can be delivered (e.g., sprayed) from the solution tank to the floor via the one or more liquid ejection holes at a predetermined speed, flow rate, duration and/or angle, depending on the surface to be cleaned. In some embodiments, a side brush may comprise a plurality of liquid ejection holes distributed on the side brush body. The liquid ejection holes may be arranged in a configuration to evenly dispense the cleaning liquid as it is delivered onto the floor. For example, in some instances, the liquid ejection holes may be spaced apart from each other in a radial direction and/or a circumferential direction of the side brush body. Liquid ejection holes that are spaced apart radially may extend radially from a center of the side brush body towards an edge of the side brush body. Conversely, liquid ejection holes that are spaced apart circumferentially may extend on the side brush body in a form of one or more concentric rings of liquid ejection holes. In some embodiments, a plurality of liquid ejection holes may be located on a spray bar attached above or in proximity to the main brush. In some embodiments, a longitudinal length of the spray bar can be substantially equal to a longitudinal length of the main brush. Cleaning liquid can be sprayed or dispensed from the spray bar onto the main brush and/or the floor ahead of the main brush, such that the main brush can clean or brush the floor with the cleaning liquid dispensed from the spray bar. The liquid ejection holes may be uniformly distributed along the spray bar. Alternatively, the liquid ejection holes may be unevenly distributed along the spray bar, such that they are closer to the side brushes and further away from the middle of the robot.

Each side brush may comprise a plurality of bristles and at least one liquid displacing component extending from the side brush body. The liquid displacing component can be configured to displace used liquid from the floor toward a predetermined direction or region as the side brush is being moved (e.g. rotated). The used liquid can be, for example the cleaning liquid (that has been sprayed via the liquid ejection holes onto the floor) which carries dirt, debris, or foreign materials after a cleaning action has been performed by the bristles.

In some instances, a liquid displacing component can comprise an elastic blade. The elastic blade may be made of rubber or any suitable elastic polymer. In some instances, the elastic blade can be detachably coupled to the side brush body, to allow for blade replacement or repair. An edge of the elastic blade may be in contact or close proximity with the floor as the robot with the side brushes is being operated. The elastic blade may be capable of bending or flexing when it contacts the floor. In some instances, the elastic blade may be located above the floor with a gap. A heigh of the gap may be, for example, about 1, 2, 3, 4, 5, 6, 7, 8, 9, 10 mm, or any value therebetween.

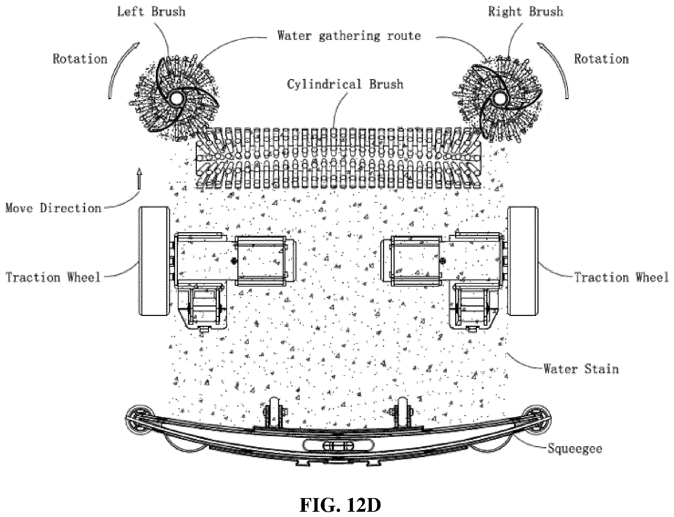

As shown in **FIG. 12B** to **FIG. 12D****,** each side brush may have a plurality of elastic blades. In some embodiments, the plurality of elastic blades (e.g., three blades) may be located equidistant from each other and extending radially from the side brush body. For example, the three blades may be oriented relative to each other such that an angle between adjacent blades is about 120 degrees. The elastic blades may be oriented in a fan-shape or propeller-like configuration. Each elastic blade can extend substantially radially from a center of the side brush body. The elastic blade can have a curved shape along a radial direction of the side brush body. The curved shape may have a curvature that is aligned with a rotating direction (clockwise or counter-clockwise) of the side brush, as shown in **FIG. 12C****.** The convex and concave surfaces of each elastic blade determine where and how used liquid is being directed or concentrated towards a target region, depending on a direction of rotation of each elastic blade. For example, when a side brush rotates in a clockwise direction (shown on the left of FIG. 12C), the elastic blades begin directing the used liquid in a counter-clockwise direction towards a center region of the side brush. Conversely, when a side brush rotates in a counter-clockwise direction (shown on the right of FIG. 12D), the elastic blades begin directing the used liquid in a clockwise direction towards a center region of the right side brush.

As shown by the arrows for the water gathering route in **FIG. 12C****,** the configuration and layout of the elastic blades allow the used liquid on the floor to be directed in a spiral manner toward a center of each side brush.

The configuration as shown in **FIG. 12B** and **FIG. 12C****,** in which the liquid displacing component is attached to the side brush, can enable the robot to remove liquid residue from an area that is being cleaned, without having or needing to extend a cleaning path width of a squeegee substantially beyond the width of the robot body. As shown in **FIG. 12D****,** the width of the squeegee may be made equal to (or slightly greater than) the width of the robot body while still being efficient in removing liquid residue from the area being cleaned, since any liquid residue that is located outside the width of the robot body can be displaced and collected by the side brush and its elastic blades, as described above. The use of the side brushes in FIGs. 12B, 12C and 12D can obviate the need to extend the width of the squeegee substantially beyond the width of the robot body, which advantageously allows the robot to maintain its compact footprint and agility, effectively clean while turning, and effectively clean dead corners.

The side brushes shown in **FIG. 12B** to **FIG. 12D** can improve cleaning performance of a robot in a variety of ways. For example, the side brushes can allow the robot to increase its scrubbing/cleaning width closer to walls. In some instances, the robot with the aforementioned side brushes may be capable of cleaning a floor region that extends as close as 1 cm to 10 cm off a wall. The elastic blades can be shaped and sized to retrieve or collect used liquid from/off the floor. In some instances, the elastic blades can remove between 60% to 100% of any liquid residue that lies within, or that extends 5% to 20% outside a footprint of the robot. Additionally, the elastic blades can be configured to prevent cleaning liquid or used liquid from splashing or extending substantially beyond a cleaning path of the robot. For example, the shape and size of each elastic blade, the orientation of the elastic blades relative to each other and relative to the bristles, the curvature of each elastic blade, among various other blade design factors, can help to reduce splashing by at least 5% to 50% compared to conventional brushes that do not have the elastic blades describe herein.

In some embodiments, the elastic blades described can be operated without requiring cleaning liquid to be sprayed or dispensed from the side brushes. In such embodiments, there is no need to direct cleaning liquid from the solution tank of the robot to the side brushes. In some instances, the side brushes (or an area proximal to the side brushes) need not have any liquid ejection holes since the elastic blades are capable of operating without cleaning liquid being dispensed from the side brushes. For example, the elastic blades described can be capable of guiding, transferring and/or spreading at least a portion of the cleaning liquid, that is dispensed from a middle bottom portion of the robot, into the area of the side brushes. This can be useful, for example to prevent excessive cleaning liquid from accumulating on the floor and spreading beyond the footprint of the robot. For example, if cleaning liquid is already being dispensed from a middle bottom portion of the robot, further dispensing of cleaning liquid from or near the side brushes may lead to excess cleaning liquid build-up, sloshing and spreading beyond the robot footprint, which can affect the cleaning performance of the robot. Accordingly, the ability to allow for the elastic blades to be operated independent of whether the side brushes are (or are not) dispensing cleaning liquid, can be a useful feature for improving cleaning performance of the robot. This can also allow the form factor and footprint of the robot to be maintained and made more compact (e.g., keeping the elastic blades within a diameter/area of the side brushes, and/or keeping the existing length of the squeegee as shown in FIG. 12D, without having to extend or increase a length or size of either the elastic blades or the squeegee). In the above-described embodiment, the elastic blades, in combination with the squeegee, can be used to collect cleaning liquid that is only dispensed from the middle bottom portion of the robot. The rotating action of the main brush can initially direct/divert the cleaning liquid away from the middle of the robot towards the side brushes for scrubbing, after which the elastic blades can be used to collect the used liquid.

In some embodiments, a ratio of (1) the cleaning liquid volume dispensed from the middle of the robot to (2) the cleaning liquid volume dispensed from the side brushes, can be dynamically adjusted by the robot to maintain or improve cleaning performance. For example, the above ratio can be adjusted to prevent excess cleaning liquid build-up, sloshing and spreading beyond the robot footprint. In some instances, the ratio of cleaning liquid dispensed (middle : side brushes) may be 10:1, 9:1, 8:1, 7:1, 6:1, 5:1, 4:1, 3:1, 2:1, or a ratio that is between any of the foregoing. In certain instances, all of the cleaning liquid is entirely dispensed from the middle of the robot, and no cleaning liquid is dispensed at all from the side brushes. In some embodiments, the liquid ejection holes on or near the side brushes may be optional. In certain cases, the side brushes (or a vicinity of the side brushes) do not have any liquid ejection holes.

### Hopper

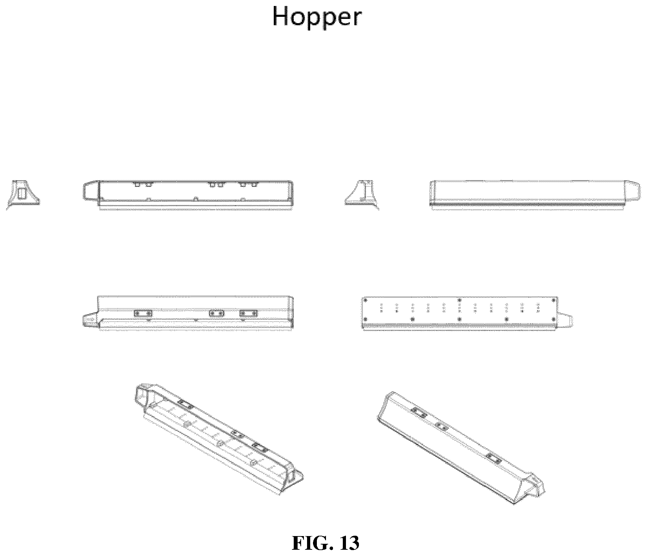

In some embodiments, the robot may comprise a hopper. In some embodiments, a hopper may be configured to collect garbage from a brush or a scrubber. In some embodiments, the hopper may comprise a sufficiently small volume such that it is easy for a human to replace, clean, empty, or refill the hopper. In some embodiments, the hopper may comprise about 0.4 L in volume. In some embodiments, a hopper may comprise at least about 0.1, 0.2, 0.3, 0.4, 0.5, 1, 2, 3, 4, or 5 L in volume. In some embodiments, the hopper may comprise at most about 0.1, 0.2, 0.3, 0.4, 0.5, 1, 2, 3, 4, or 5 L in volume. As shown in **FIG. 12****,** in some embodiments, the hopper may be configured to magnetically attach to the brush deck of the robot brush subsystem. In some embodiments, the hopper may be configured to releasably attach to the brush deck of the robot brush subsystem. In some embodiments, the hopper may be configured to detach from the brush deck of the robot brush subsystem. **FIG. 13** illustrates one non-limiting example of the physical hardware configuration for the hopper.

In some cases, the hopper may be positioned behind one or more brushes of the robot. In some cases, the hopper may be configured to receive and/or collect pieces of debris or foreign materials that have been directed to the hopper by the one or more brushes. For instance, the hopper may comprise an opening toward the one or more brushes, through which the debris or foreign materials are introduced into the hopper. An lower edge of the opening of the hopper may maintain a close contact with the floor, such that even small pieces of the debris or foreign materials are collected into the hopper. In an example, the lower edge of the opening of the hopper may comprise an elastic blade which is in contact with the floor.

In some embodiments, the hopper may be configured to receive a solid garbage. In some embodiments, the hopper may be configured to receive a mixture of solid garbage and water content and filter the water content out. The mixture of solid garbage and water content can be produced from the main brush as a result of the cleaning liquid sprayed or dispensed from the spray bar, as described elsewhere in the disclosure. For example, a bottom surface of the hopper may comprises a plurality of drain holes. A size of the drain holes can be selected to pass out a water content while keeping the solid garbage within the hopper. The water content may then be picked up and removed by the squeegee, as described elsewhere in the disclosure.

### Tank

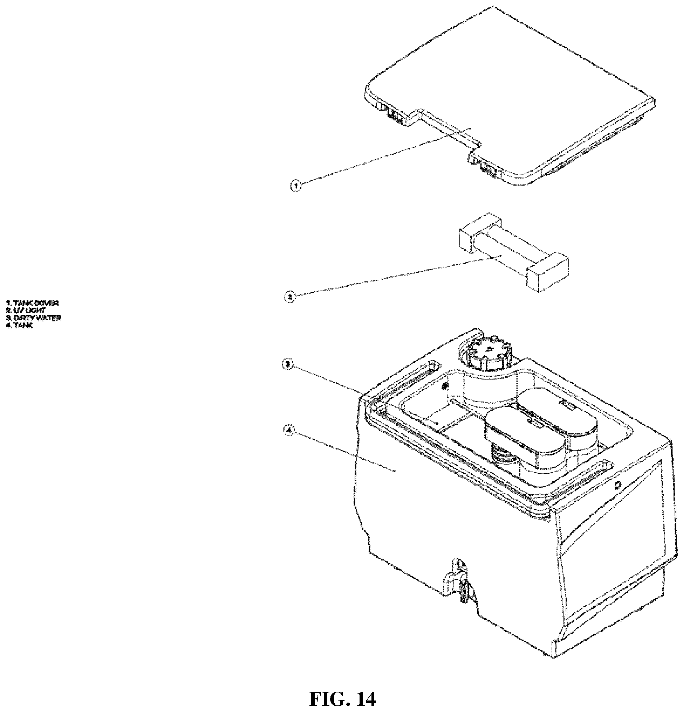

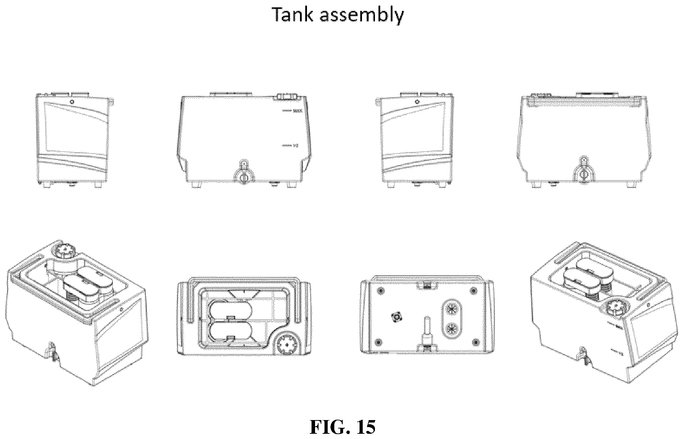

In some embodiments, the robot may comprise a tank provided within the chassis of the robot. **FIG. 14** and **FIG. 15** schematically illustrate an example of a tank that may be integrated within a chassis of the robot. The tank may comprise a tank for holding water and/or solution or detergent, and a cover for the tank. In some cases, the tank may comprise a UV light for sanitizing or disinfecting waste water in the tank. In some cases, the tank may be positioned at or near a top portion of the robot such that the top of the robot is heavier than the bottom of the robot. The placement of the tank at or near the top portion of the robot can make it easier for a user to access or service the tank. Alternatively, the tank may be positioned at or near a bottom portion of the robot such that the bottom of the robot is heavier than the top of the robot.

In some embodiments, the tank integrates a solution tank and a waste water tank. In some embodiments, the tank comprises a solution tank integrated with a waste water tank. In some embodiments, the liquid in the solution tank is substantially evenly sprayed onto the rolling brush and/or the side brushes for cleaning the ground. In some embodiments, a vacuum is generated in the waste water tank. In some embodiments, the vacuum is generated using a water suction motor assembly. In some embodiments, a negative pressure is generated in a water suction pipe to collect the waste water on the ground back to the waste water tank. In some embodiments, a foldable handle is provided on top of the cleaning machine. In some embodiments, a front side of the cleaning machine is provided with sensors such as radar, binocular camera, TOF, or any combination thereof.

In some embodiments, the robot may comprise a tank. In some embodiments, the tank may comprise a sufficiently small volume such that it is easy for a human to replace, clean, empty, or refill the tank. In some embodiments, the tank may comprise about 10 L or 11 L in volume. In some embodiments, a tank may comprise at least about 5, 6, 7, 8, 9, 10, 11, 12, 13, 14, 15, 16, 17, 18, 19, or 20 L in volume. In some embodiments, a tank may comprise at most about 5, 6, 7, 8, 9, 10, 11, 12, 13, 14, 15, 16, 17, 18, 19, or 20 L in volume. In some embodiments, a tank may be a recovery tank, a solution tank, or any other tank for holding a liquid.

In some embodiments, the robot may comprise at least two spatially separated tanks. In some embodiments, the at least two spatially separated tanks may be adjacently separated by a partition. In some embodiments, the at least two spatially separated tanks may be non-adjacently separated. In some embodiments, the at least two spatially separated tanks may be configured to prevent or reduce cross-contamination between contents of the at least two tanks. In some embodiments, the at least two tanks may comprise elevated spouts that are configured to prevent or reduce cross-contamination between contents of the at least two tanks. In some embodiments, one of the at least two spatially separated tanks may be a tank for holding water and/or cleaning solution. In some embodiments, one of the at least two spatially separated tanks may be a tank for holding dirty water or used cleaning solution. In some embodiments, the tank for holding the water and/or cleaning solution may comprise a cap or a spout of a first distinct shape or color. In some embodiments, the tank for holding the dirty water or the used cleaning solution may comprise a cap or a spout of a second distinct shape or color. In some embodiments, the first distinct shape or color may be different from the second distinct shape or color. In some embodiments, the first distinct shape or color may be different from the second distinct shape or color such that a user can tell which tank is for holding the water and/or cleaning solution versus dirty water or used cleaning solution. In some embodiments, the tank may comprise a UV disinfectant system operably coupled thereto. In some embodiments, the tank for holding the dirty water or used cleaning solution may comprise a UV disinfectant system operably coupled thereto.

In some embodiments, the robot may comprise a solution tank as described elsewhere herein. The solution tank may be configured to hold both clean water and a cleaning solution. In some cases, a user may fill the solution tank with clean water and add cleaning detergent. In some cases, the solution tank may be (a) filled with a pre-mixed cleaning solution or (b) filled with clean water that the robot can automatically mix cleaning detergent with at or near the point of dispensing the cleaning solution onto the floor.

### Squeegee

In some embodiments, a robot may comprise a squeegee. The squeegee may be used to clean or remove waste water or water residue from the floor being cleaned. In some embodiments, a negative pressure can be applied to collect the waste water from the floor back to the waste water tank. As described elsewhere in the disclosure, cleaning water can be sprayed or dispensed onto the brush and/or the floor ahead of the brush, such that the debris or foreign materials may be removed from the floor with a friction between the brush and the floor. A mixture of the debris or foreign materials and the cleaning water may subsequently be collected into the hopper. The water content in the mixture may be filtered out from a bottom of the hopper onto the floor. The water content (e.g., waster water) may then be collected by the squeegee and transferred from the floor to the waste water tank with the aid of a negative pressure.

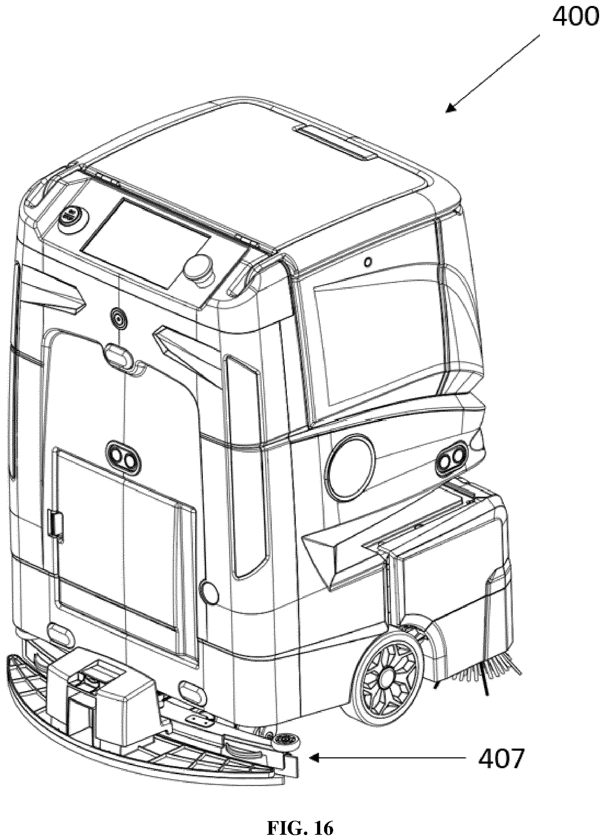

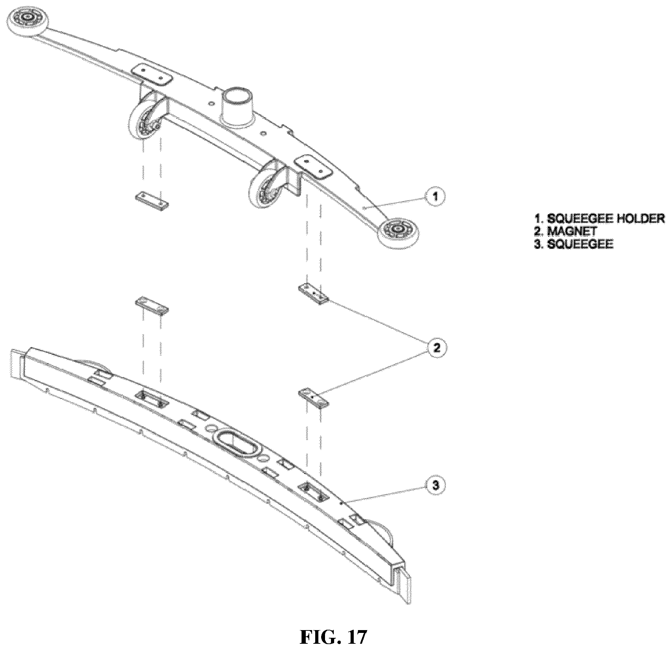

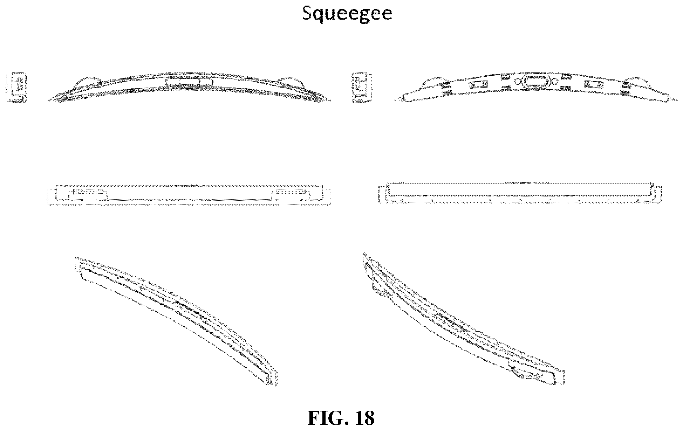

**FIG. 10** and **FIG. 16** schematically illustrate a robot 400 comprising a squeegee 407 that is attachable to the chassis of the robot 400. The squeegee 407 may be located in front of a dry mop of the robot. As shown in **FIG. 17** and **FIG. 18****,** in some cases, the squeegee may be configured to releasably attach to a holder (e.g., using one or more magnets). In some embodiments, the squeegee may be configured to releasably couple to the robot via the holder. In some cases, the holder may comprise one or more wheels for aiding or guiding a movement of the squeegee across a surface to be cleaned as the robot moves across the surface.

In some embodiments, the squeegee component comprises a consumable part. In some embodiments, the squeegee may comprise a consumable part that is convenient and quick for detachment and/or installation.

In some embodiments, a robot may comprise a squeegee capable of quick replacement without a tool. In some embodiments, the squeegee may be an integrated squeegee component.

In some embodiments, the squeegee may comprise a front squeegee strip. In some embodiments, the squeegee may comprise a rear squeegee strip. In some embodiments, the front squeegee strip and the rear squeegee strip comprise a rubbery material.

In some embodiments, the squeegee comprises a frame. In some embodiments, the frame comprises a plastic material.

In some embodiments, the integrated squeegee component comprises at least 1 or 2 rubber pads. In some embodiments, a rubber pad comprises a mounting hole.

In some embodiments, the robot may comprise at least 1 or 2 ball head mount rods. In some embodiments, the ball head mount rod may be provided on a bracket on the robot. In some embodiments, the ball head meant rod may couple with the squeegee component. In some embodiments, the diameter of the ball head is larger than the mounting hole of the rubber pad. In some embodiments, when installing and removing the squeegee component onto and from the cleaning robot, the ball head is squeezed into the mounting hole of the rubber pad to realize a quick detachment and installation without a tool. In some embodiments, when installing the squeegee component onto the cleaning robot, an operator may align the ball head with the mounting hole of the rubber pad, and push the ball head into the mounting hole of the rubber pad. In some embodiments, when detaching the squeegee component from the cleaning robot, an operator may press the squeegee component down from the mounting bracket on the cleaning robot without needing a specific tool.

### Handle

In some embodiments, the robot may comprise a handle for operating, pushing or carrying the robot. The handle can be used to engage an operational mode, to control the robot in a selected mode, or to switch between different operational modes.

In some embodiments, in an operating state, the brush assembly and the squeegee assembly are lowered to the ground. In some embodiments, when the handle is opened, the cleaning machine is in a manual operation mode. In some embodiments, in the manual operation mode, a cleaning operation or a map constricting operation can be performed. In some embodiments, when the handle is closed, the cleaning machine is in an automatic operation mode.

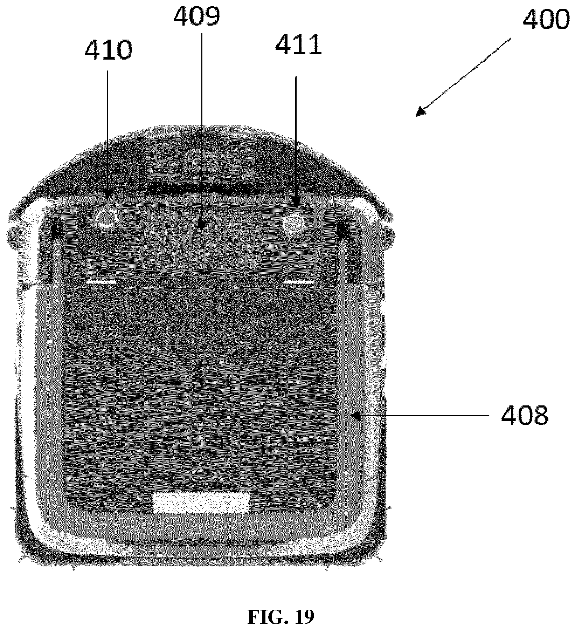

**FIG. 19** shows a top portion of the robot 400, which may comprise a handle 408 for operating the robot 400. The handle 408 may be movable between two or more positions. The two or more positions may correspond to different operating modes. In some cases, the handle 408 may be flush with a top surface of the robot. In some cases, the handle 408 may be angled relative to a top surface of the robot. In some cases, the handle may be temporarily locked at a given position to allow an operator to utilize the robot in a desired operating mode without the need to constantly monitor or control the position of the handle relative to the robot.

### Cleaning Detergent Distribution Subsystem

In some aspects, the present disclosure provide a structure for distributing cleaning detergent. In some embodiments, the structure may comprise a substantially circular detergent balls. In some embodiments, the circular detergent balls may automatically drop into the water tank. In some embodiments, the circular detergent balls may automatically drop into the water tank by gear rotation transmission, a spring, gravity, or any combination thereof. In some embodiments, the detergent balls may be configured to dissolve in water in less than about one minute, to create detergent water. In some embodiments, the structure may comprise a detergent sheet. The detergent sheet may be paper-thin, dissolvable sheets that have all the necessary ingredients to create detergent water. The detergent sheet may automatically drop into the water tank. In some embodiments, the detergent sheet may be configured to dissolve in water in less than about 20 seconds to create the detergent water.

In some embodiments, a user may manually engage a button on the robot (e.g., pressing or rotating a button). In some embodiments, engaging the button may result in at least one of a plurality circular detergent balls in a funnel container dropping into the water tank (e.g., through a rotation of a gear and gravity). In some embodiments, the circular detergent ball dissolves in the water in less than about one minute, turning the water in the water tank into detergent liquid. In some embodiments, the detergent liquid is automatically sprayed onto the ground. In some embodiments, the user can determine the number of detergent balls to be added in the water tank based at least in part on a cleanness of the ground.

### Bumper

In some embodiments, the robot may comprise a bumper. In some embodiments, the robot may comprise a bumper assembly. In some embodiments, the bumper assembly may comprise a bumper. In some embodiments, the bumper may be located in front of one or more drive wheels of the robot. In some embodiments, the bumper assembly may comprise a bumper support. In some embodiments, the bumper support may be configured to support the bumper. In some embodiments, the robot may comprise a first cage (e.g., at an upper relative location), a second cage (e.g., at a lower relative location). In some embodiments, the first cage, the second cage, or both may be installed on the chassis. In some embodiments, one or more balls may be installed in the cages. In some embodiments, the robot may comprise one or more micro switches. In some embodiments, the one or more micro switches may comprise two or more micro switches (e.g., one on the left side and the other one on the right side in a lateral direction). In some embodiments, the one or more micro switches may be installed on the chassis. In some embodiments, the robot may comprise one or more levers. In some embodiments, the one or more levers may comprise two or more levers. In some embodiments, the one or more levers may be installed on the chassis. In some embodiments, the lever may comprise or be operably connected to a restoring spring. In some embodiments, the bumper or the bumper assembly may be configured to actuate a micro switch when bumped. In some embodiments, the micro switch may be configured to stop the robot when actuated. For example, if the robot collides with an obstacle in front of the robot during an autonomous operation, the bumper can be displaced within a predetermined stroke and the micro switch can be actuated, such that the robot can be stopped for safety. In some embodiments, a robot may be equipped with a radar, a camera, or both. In some embodiments, the radar, the camera, or both may be used for navigating the robot. In some embodiments, the radar, the camera, or both may be used for detecting obstacles. In some cases, a robot comprising the bumper or the bumper assembly may be advantageous to a robot having only vision or sound sensors, because the bumper or the bumper assembly may provide obstacle detection where the vision or sound sensors may have blind spots. For example, during an autonomous operation of the robot, the robot is not able to stop if it is unable to detect an obstacle in a blind spot.

In some cases, the bumper system can be equipped with auto backward movement and switch off functions when the machine is close to an obstacle in emergency situation where the reaction time is not enough for it to move around an object.

### Magnetic Attachment

In some cases, the robot may comprise an attachment mechanism for attaching various components to the robot. The attachment mechanism may comprise, for example, a magnetic attachment. The magnetic attachment may enable attachment of a brush, a squeegee, a bumper, a hopper, or any other component of the robot on the body or chassis of the robot.

### Treatment and Sanitization Subsystem

In some embodiments, the robots or machines may comprise a treatment or sanitization subsystem. The treatment or sanitization subsystem may be configured to perform a treatment operation (e.g., a sanitization operation or a disinfection operation) for one or more components or portions of the robot or machine. In some cases, the treatment or sanitization subsystem may be configured to treat or sanitize a hazardous or toxic material or any other material that can be harmful to human health.

In some cases, a gas or a fluid may pass through a portion of the robot or machine to perform a cleaning operation. The gas may comprise, for example, ambient air that can be sucked into the robot or machine from the environment surrounding the robot or machine (e.g., by way of a vacuum or a negative pressure source). The fluid may comprise, for example, water, cleaning fluid, or any mixture thereof. In some cases, the gas or fluid may be used to pick up or transport dirt, debris, or other unclean objects or substances from an environment or area to be cleaned. In some cases, the gas or fluid may be used to remove dirt, debris, or other unclean objects from an environment or area to be cleaned. In some cases, the gas or fluid may serve as a substrate which can carry debris, and the gas or fluid may be sucked into the cleaning machine (e.g., by using a negative pressure source). Alternatively or additionally, the gas or fluid may serve as a sanitizing material or substance.

In some embodiments, the gas or fluid may pass through one or more components or subsystems of the robot or machine (e.g., a filter or a recovery tank), and the one or more components or subsystems may build up dirt, debris, or other unclean objects or substances over time (either naturally over the course of machine operation, or due to insufficient cleaning of the components or subsystems by an operator of the robot or machine). This can generate an odor, and in some cases, the dirt, debris, or other unclean objects or substances can be hazardous, toxic, or otherwise harmful to human health. In such a scenario, the treatment or sanitization subsystem described above can be used to perform a treatment procedure and/or to at least partially sanitize or disinfect the dirt, debris, or other unclean objects or substances collected during a cleaning operation in order to make the dirt, debris, or other unclean objects or substances less hazardous, toxic, or harmful. The treatment or sanitization process may optionally involve treatment of waste water to reduce biohazard exposure and to mitigate health risks to operators.

In some cases, the treatment or sanitization subsystem can be integrated with or embedded in a component or another subsystem of the robot or machine. The component or other subsystem of the robot or machine may comprise, for example, a recovery tank of the robot or machine. In some cases, the treatment or sanitization subsystem can be provided on or coupled to an external portion of the robot or machine for ease of access, servicing, or maintenance. In some cases, the treatment or sanitization subsystem may be activated when the recovery tank is closed.

In one non-limiting example, the treatment or sanitization subsystem can comprise a light source configured to irradiate a material with light in order to perform a disinfection or sanitization process. In some cases, the light source may be attached to or integrally formed on an inside portion of a recovery tank of a cleaning robot or a machine.

In some cases, the light source may comprise a UV light source. The UV light source may comprise one or more UV-LEDs. The UV light source may emit light having a wavelength corresponding to ultraviolet (UV) light. In some cases, the treatment or sanitization subsystem may comprise a UV light or a UV-C light having potent germicidal properties (e.g., the ability to inactivate viruses and bacteria). In some cases, the wavelength of light may range from about 100 nanometers (nm) to about 280 nm. Exposure to the light may result in sanitization or disinfection, or even sterilization depending on how much UV energy is emitted.

In some cases, the UV light source may be powered by a power supply located on the robot or machine. In some cases, the UV light source can be powered by a solar panel. The solar panel may comprise one or more solar energy cells. In some embodiments, a solar panel and/or a power source can be electrically coupled to the UV light source to provide power to the UV light source (e.g., at different times during the day).

As described above, the UV light source may comprise one or more UV-LEDs. In some cases, the one or more UV-LEDs may be provided in a grid pattern. Alternatively, the UV-LEDs may be provided in a radial pattern, or any regular or irregular pattern. In some cases, a plurality of UV-LEDs may be controlled, either individually or collectively, to illuminate a component or an internal area or volume of the robot or machine for cleaning, treatment, sanitization, and/or disinfection.

In some embodiments, the robots or machines described herein may be configured to monitor the cleanliness level of the waste water or recovery tank. In some cases, the robots or machines may be configured to activate a UV light periodically to help sanitize, disinfect, or deodorize the tank after it has been emptied. In some cases, the robots or machines may monitor the cleanliness of the tank inside the robot or machine which is used to recover waste water, which can contain contaminants, dirt, used cleaning solution, and other harmful or undesirable materials or substances. In some embodiments, a tank disinfecting, sanitization, or de-odorizing cycle can be activated after the conclusion of every cleaning operation, as long as the tank has been emptied of waste water. In some embodiments, a tank disinfecting, sanitization, or de-odorizing cycle can be activated based on the floor area cleaned or the duration of cleaning. In some embodiments, a tank disinfecting, sanitization, or de-odorizing cycle can be activated based on a measurement of the dirtiness level of the water that is in the waste water tank, or the dirtiness level of the waste water tank once it has been emptied. In some instances, a UV light may be used for the tank disinfecting, sanitization, or de-odorizing cycle. If a UV light is used to disinfect or sanitize the interior of a waste water tank or a recovery tank, then the tank may be lined with a metallic material, since UV light can cause damage to plastic linings.

### Filter

In some cases, the robot may comprise one or more filters. The filters may comprise a high efficiency particulate air (HEPA) filter. The filters may be configured to capture dust or dirt particles that are flowing into or through the robot (e.g., when the robot utilizes a vacuum or another negative pressure source to collect dust or debris).

### Robot Operation

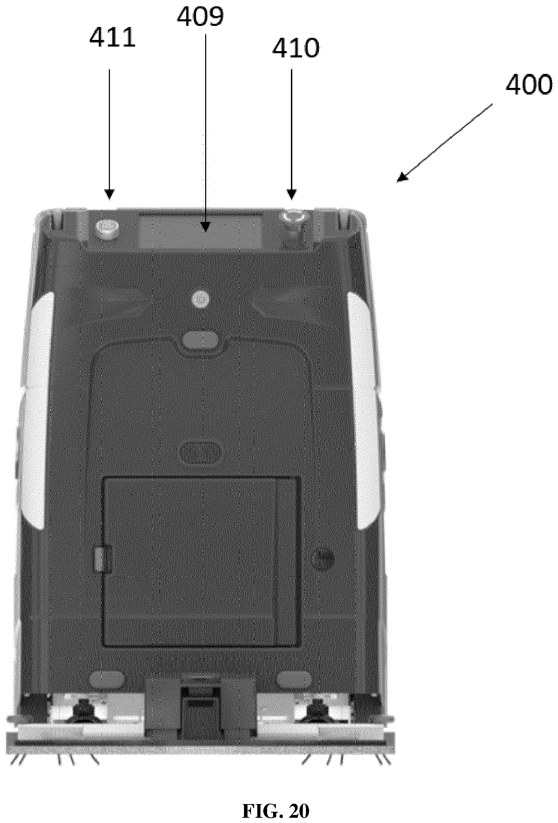

Referring back to **FIG. 19** and **FIG. 20****,** in some embodiments, the robot 400 may comprise a screen 409 integrated with a body or a chassis of the robot 400. The screen 409 may be configured to provide a user interface for navigating, controlling, teaching, training, or otherwise operating the robot. In some cases, the robot may comprise a first button 410 and a second button 411. The first button 401 and/or the second button 411 may be used to initiate or to stop a cleaning operation or procedure.

### Mapping / Navigation

In some embodiments, the robot may be autonomously operated based at least partially on a map. In some embodiments, the map may be constructed using a mapping method. In some embodiments, the mapping method may be constructed based at least partially on a user input. In some embodiments, the user input may comprise pushing the robot using a handle. In some embodiments, the mapping method may comprise using a simultaneous localization and mapping (SLAM) or visual SLAM (VSLAM) method. In some embodiments, the mapping method may comprise using sensor data from one or more sensors. In some embodiments, the one or more sensors may be disposed on the robot. In some embodiments, the one or more sensors may be fused. In some embodiments, the mapping method may comprise calculating a movement trajectory and/or position information of the robot.

In some embodiments, the mapping method may comprise opening a handle of the robot. In some embodiments, the mapping method may comprise scanning a position calibration code using a vision sensor and/or a navigation sensor. In some embodiments, the calibration code may comprise a QR code or a barcode. In some embodiments, the mapping method may comprise pushing the handle. In some embodiments, pushing the handle may initiate calculation of a trajectory and/or position information of the robot. In some embodiments, the calculation may be based at least partially on data (e.g., VSLAM data) collected through one or more vision and/or one or more navigation sensors configured or implemented for SLAM or VSLAM applications. In some embodiments, the method may comprise releasing the handle. In some embodiments, the method may comprise scanning the position calibration code a second time. In some embodiments, scanning the position calibration code the second time saves the map on a digital storage device.

### Sensors

The systems disclosed herein may comprise one or more sensors. The one or more sensors may comprise one or more vision sensors and/or one or more navigation sensors. The one or more vision sensors may be configured to create a visualization of the robot's surrounding environment, or otherwise simulate or model the surrounding environment based on data obtained using the one or more sensors. The one or more navigation sensors may be used to obtain data that can be used by the robot to navigate a surrounding environment or travel along a path while avoiding obstacles. In some non-limiting embodiments, the one or more sensors may comprise a binocular camera, a radar unit, a time-of-flight (TOF) camera, a LIDAR unit, an ultrasonic or infrared sensor, a cliff sensor that utilizes ultrasonic or infrared waves, an inertial unit (e.g., an inertial measurement unit or IMU), an accelerometer, a velocity sensor, an impact sensor, a position sensor, a GPS, a gyroscope, an encoder, an odometer, or any other type of sensor as described elsewhere herein.

In some embodiments, the one or more sensors may be configured to obtain operational data for a robot or a machine. In some embodiments, the one or more sensors may be configured to obtain data about an environment in which the robot or machine is operating, or one or more objects in the environment. The one or more objects may comprise stationary objects or moving objects.

In some embodiments, the one or more sensors may comprise, for example, a wheel sensor, an encoder, or a clock or a timing unit for measuring machine or component operational time.

In some embodiments, the one or more sensors may comprise an ATP sensor. In some cases, the one or more sensors may be configured to sense a type of dirt being cleaned, detect the presence of a bacteria, virus, or pathogen in a target area or the air surrounding the target area, and/or determine a type of bacteria, virus, or pathogen. In some embodiments, the one or more sensors may comprise an air quality sensor.

In some embodiments, the one or more sensors may comprise a vision sensor (e.g., a computer vision sensor) and/or a navigation sensor. The vision sensor and/or the navigation sensor may comprise a lidar unit, a time of flight (TOF) camera, a binocular vision camera, or an ultrasonic sensor. In some cases, the vision sensor and/or the navigation sensor may be configured to detect debris on a floor. In some cases, a processor that is operatively coupled to the vision sensor and/or the navigation sensor may be configured to reprioritize a robot's route, a machine's route, or a cleaning routine / logic to pick up the remaining debris or to minimize the amount of debris remaining after a cleaning operation. In some cases, the one or more visions sensors and/or the one or more navigation sensors can be used to detect water on a floor or water remnants / residue, and navigate the robot towards or around the water remnants / residue.

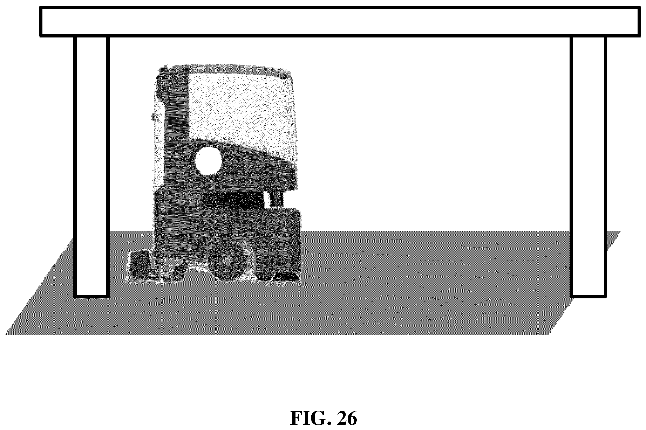

In some cases, the one or more vision sensors may be configured to detect a change in an amount or intensity of light in a cleaning environment. For example, **FIG. 26** shows a robot that is moving under a structure. While moving under the structure, the lighting conditions may change, and the robot may change or adjust its operation based on the detected changes in the lighting conditions. In some cases, the robot may travel to a location that has more or less light than another location. When the sensors detect a change in the lighting conditions, the robot may utilize one or more other sensors to continually sense objects in the vicinity, navigate seamlessly along a cleaning path, and/or dynamically adjust its route.

In some embodiments, the one or more sensors may comprise an impact sensor or an accelerometer that is configured to sense impacts and abuse. The impact sensor or accelerometer can be used to measure and report the force of the impact and sense abnormal impacts. In some cases, the data obtained using the impact sensor or accelerometer can be provided to a processing unit. In some cases, the processing unit may be configured to generate one or more signals based on the processing of said data. The one or more signals may correspond to an instruction to send a push notification for an impact event, or to notify an operator of the impact event via a display or a screen.

In some embodiments, the one or more sensors may be configured to detect amp draw for one or more motors of a cleaning machine or robot. In some cases, the one or more sensors can also detect changes or variations in amp draw over time, which can indicate sub-optimal robot or machine operation or usage.

In some cases, the one or more sensors may be configured to obtain one or more sensor readings. The one or more sensor readings may indicate the cleanliness of waste water collected by the machine, or the quality of air of the environment. In some cases, the quality of the air may be determined based on a cleanliness of a filter used to capture and filter particles from the air of the environment in which a cleaning robot or machine is operated. In some non-limiting embodiments, the one or more sensor readings may be derived using a total dissolved solids (TDS) sensor.

In some embodiments, the one or more sensors may be configured to measure light reflectivity of a surface. The measurements of light reflectivity may indicate the relative cleanliness of the surface. The measurements of light reflectivity may be taken before and after cleaning and compared to determine a change in a reflectivity of the surface due to a cleaning operation.

In some embodiments, the one or more sensors may be configured to obtain operational data for a robot or machine. In some embodiments, the operational data may comprise information on a frequency at which one or more treatment or sanitization procedures occur or need to occur. In some cases, the operational data may comprise information on a duration for which treatment or sanitization procedures occur or need to occur.

In some embodiments, the one or more sensors may comprise one or more vision sensors as described above. In some cases, the vision sensors may be configured to detect debris on a floor. In some cases, the vision sensors may be configured to detect the amount or level of water remaining on a floor or a surface.

In some embodiments, the one or more sensors may comprise an impact sensor or an accelerometer as described above. The impact sensor or accelerometer can be used to determine cleaning efficiency, and whether the robot, machine or an operator of the robot or machine has followed an optimal path or cleaning routine (i.e., whether the actual path or cleaning routine used corresponds to a trained or reference path or cleaning routine).

In some embodiments, the one or more sensors may comprise one or more sensors that can detect pollutants or particles in the air. In some cases, the one or more sensors can be configured to detect a concentration of the pollutants or particles relative to other particles present in the air.

### Operational Data

The operational data of one or more robots or machines in a fleet may be gathered or obtained using one or more sensors of the one or more robots or machines. In some cases, the one or more sensors may comprise one or more vision sensors and/or one or more navigation sensors as described elsewhere herein. In some cases, the one or more sensors may comprise a position sensor, a GPS unit, an encoder, an odometer, an accelerometer, an inertial measurement unit (IMU), a gyroscope, or a velocity sensor. In some cases, the one or more sensors may comprise, for example, a temperature sensor, a pressure sensor, a humidity sensor, or any other type of environmental sensor for sensing the conditions of the environment in which the one or more robots or machines are being operated. In some cases, the one or more sensors may comprise an optical sensor or a vision sensor as described elsewhere herein. The optical sensor or vision sensor may comprise, for example, an imaging sensor or a camera. In some cases, the one or more sensors may comprise a lidar sensor, a vision sensor, a time of flight sensor (e.g., a 3D time of flight sensor), a binocular vision sensor, a stereoscopic vision sensor, or an ultrasound sensor.

In some embodiments, the operational data may be received from a single robot and/or machine or from multiple robots and/or machines. In some cases, the operational data may be received from multiple robots and/or machine in series or sequentially. Alternatively, the operational data may be received from multiple robots and/or machines simultaneously or concurrently. As described above, the robots and/or machines may comprise autonomous, semi-autonomous, and/or non-autonomous robots or machines, or any combination thereof. Any combination of robots and/or machines, including autonomous, semi-autonomous, and non-autonomous machines or robots, can be used together to implement the systems and methods of the present disclosure.

In some cases, the operational data may comprise information on a geographical location of the one or more robots or machines. In some cases, the operational data may comprise information on a position, an orientation, or a pose of the one or more robots or machines. In some cases, the operational data may comprise information on a spatial distribution of the one or more robots or machines across an area or an environment.

In some cases, the operational data may comprise information on a battery level or a charge status of the one or more robots or machines and/or the one or more components of the one or more robots or machines. The battery level or charge status may indicate how long the robot or machine has been in operation, and how long the robot or machine may continue operating before losing power.

In some cases, the operational data may comprise fault information or alarm information for the one or more robots or machines and/or the one or more components of the one or more robots or machines. In some cases, the fault information may be generated automatically by the one or more robots or machines. In some cases, the fault information may be manually reported or generated by a user or an operator of the one or more robots or machines.

In some cases, the operational data may comprise information on work records, a cleaning path, or a cleaning performance for the one or more robots or machines. In some cases, the operational data may comprise information on a total time of use or operation for the one or more components.

In any of the embodiments described herein, the operational data may be periodically generated or compiled by the one or more robots or machines for transmission or upload to the central server. In any of the embodiments described herein, the operational data may be transmitted from the one or more robots or machines to the central server at one or more predetermined or periodic time intervals. In any of the embodiments described herein, the operational data may be transmitted from the one or more robots or machines to the central server at one or more time intervals that vary according to a historical usage or a total operational time of the one or more robots or machines.

In some embodiments, the operational data may be obtained using a float sensor. In some cases, the float sensor can indicate a full recovery tank and alert a user that the tank needs to be changed. In some cases, the float sensor can indicate an empty solution tank and alert a user that the tank needs to re-filled.

In some embodiments, the operational data may comprise information on an operational time of the robot or machine. The information on the operational time of the robot or machine can be used to determine when to activate a treatment or sanitization subsystem as described elsewhere herein. In some cases, the information on the operational time of the robot or machine can be used to alert or inform a user as to when the user should initiate a treatment or sanitization procedure (e.g., to sanitize or clean a component or subsystem of the robot or machine, or to disinfect a harmful substance or byproduct that is generated or built up over time as the robot or machine performs one or more cleaning operations).

In some embodiments, the operational data may comprise information on a frequency at which treatment or sanitization procedures occur or need to occur. In some cases, the operational data may comprise information on a duration for which treatment or sanitization procedures occur or need to occur. In some cases, the frequency information and/or the duration information may indicate how much or how often cleaning is performed over time.

In some embodiments, the cleaning data or information can be used to identify water spots or other spots that may require additional cleaning, and to change the operation of the machine or the components of the machine to optimize cleaning performance.

In some cases, the cleaning data or information may comprise information on environmental factors associated with an operating environment of the robot or machine. The environmental factors may include, for example, temperature, humidity, or area of operation. In some cases, for example in colder climates, the robot or machine may automatically adjust its operation or movement to operate slower, increase vacuum power, and/or increase water flow.

### Exploded View Figures

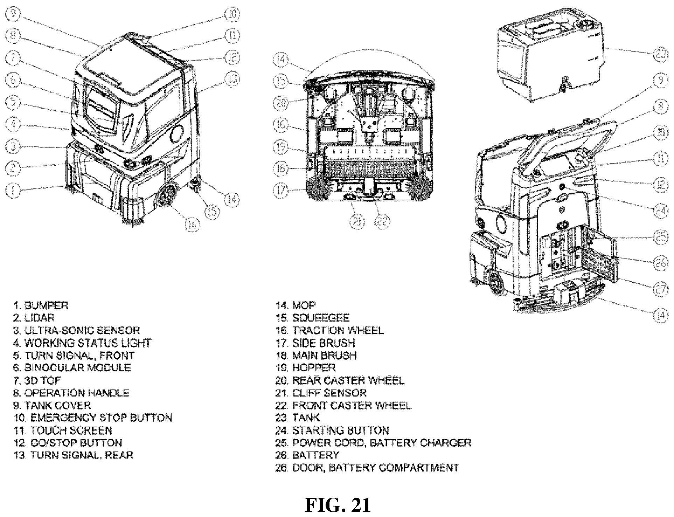

**FIG. 21** illustrates an example of a robot system. In some cases, the robot system may comprise a bumper. The bumper may be positioned on an outer portion of the robot to absorb impacts and reduce the risk of damage to internal components of the robot.

In some case, the robot system may comprise one or more sensors. The sensors may comprise, for example, LIDAR, an ultrasonic sensor, a binocular vision camera, a time-of-flight camera, or a cliff sensor.

In some case, the robot system may comprise one or more status indicators (e.g., Working Status Light). In some case, the robot system may comprise one or more turn signals. In some cases, the one or more turn signals may be disposed on a front portion of the robot system. In some cases, the one or more turn signals may be disposed on a back portion or a side portion of the robot system.

In some cases, the robot system may comprise an operation handle. The operational handle may be used to control the robot or select an operational mode for the robot.

In some cases, the robot system may comprise a tank. In some cases, the robot system may comprise a tank cover.

In some cases, the robot system may comprise one or more buttons (e.g., an emergency stop button, a start button, a go/stop button. In some cases, the robot system may comprise a touch screen.

In some cases, the robot system may comprise one or more cleaning tools. In some cases, the robot system may comprise a mop, a squeegee, a main brush, a side brush, or any combination thereof.

In some cases, the robot system may comprise one or more wheels. In some cases, the robot system may comprise a traction wheel, a rear caster wheel, a front caster wheel, or any combination thereof.

In some cases, the robot system may comprise a hopper. The hopper may comprise any configuration as described elsewhere herein.

In some cases, the robot system may comprise a power cord. In some cases, the robot system may comprise a power cord with a battery charger. In some cases, the robot system may comprise a battery. In some cases, the robot system may comprise a battery compartment. In some cases, the robot system may comprise a door for the battery compartment.

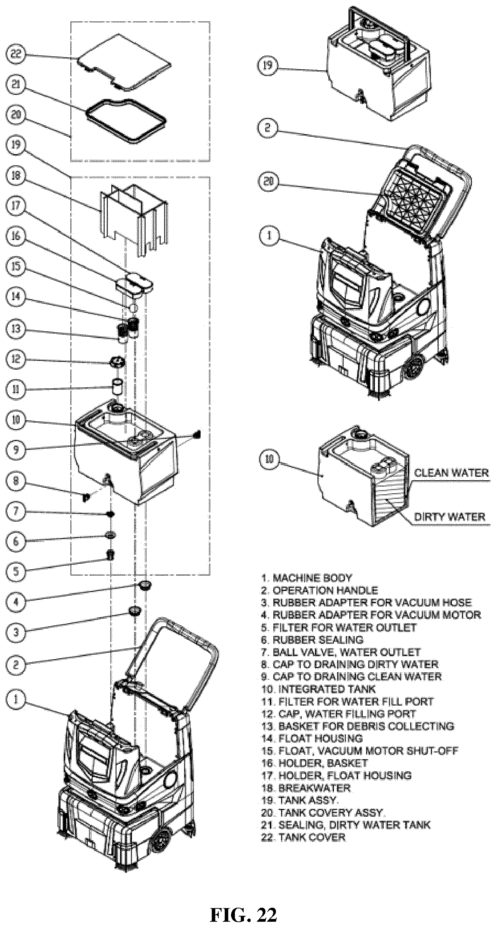

**FIG. 22** illustrates an example of a tank subsystem of the robot. In some cases, the tank subsystem may be disposed in a body or chassis of the robot. In some cases, the tank subsystem may comprise a tank. In some cases, the tank subsystem may be accessible when an operation handle of the robot is lifted or raised.

In some cases, the tank subsystem may comprise one or more rubber adapters. In some cases, the one or more rubber adapters are configured to create a vacuum seal. In some cases, the one or more rubber adapters are configured to connect with a vacuum hose. In some cases, the one or more rubber adapters are configured to connect with a vacuum motor. In some cases, the tank subsystem may comprise a water outlet. In some cases, the tank subsystem may comprise a filter for a water outlet. In some cases, the tank subsystem may comprise one or more rubber sealings. In some cases, the one or more rubber sealings may be configured to prevent leakage of a fluid into our out of the tank.

In some cases, the tank subsystem may comprise one or more valves. In some cases, the one or more valves may be configured to control a flow of water into our out of the tank subsystem. In some cases, the one or more valves may be configured to control a flow of water through the water outlet.

In some cases, the tank subsystem may comprise one or more caps. In some cases, the one or more caps may be configured to, when opened, drain dirty water. In some cases, the one or more caps may be configured to, when opened, drain clean water. In some cases, the tank subsystem may comprise a water filling port. In some cases, the water filling port may comprise a cap.

In some cases, the tank subsystem may comprise a filter. In some cases, the filter may be for filtering water input through the water filling port.

In some cases, the tank subsystem may comprise a basket for collecting debris. In some cases, the tank subsystem may comprise a holder for the basket.

In some cases, the tank subsystem may comprise a float. In some cases, the float may be configured to shut-off vacuum flow. In some cases, the tank subsystem may comprise a housing for the float. In some cases, the tank subsystem may comprise a float housing. In some cases, the tank subsystem may comprise a holder for the float housing.

In some cases, the tank subsystem may comprise a cover. The cover may be disposed on a top portion of the tank subsystem, and may be removable by an operator to access the tank subsystem or any components of the tank subsystem.

In some cases, the tank subsystem may comprise a solution tank. In some cases, the tank subsystem may comprise a dirty water tank. In some cases, the tank subsystem may comprise a seal for the dirty water tank.

In some embodiments, the robot may comprise a brush and hopper subsystem. In some cases, the brush and hopper subsystem may be configured to interface with a chassis of the robot.

In some cases, the brush and hopper subsystem may comprise one or more brushes. In some cases, the brush and hopper subsystem may comprise one or more drivers for the one or more brushes. In some cases, the brush and hopper subsystem may comprise a side brush. In some cases, the brush and hopper subsystem may comprise a drive for the side brush. In some cases, the brush and hopper subsystem may comprise a main brush. In some cases, the brush and hopper subsystem may comprise a drive for the main brush. In some cases, the brush and hopper subsystem one or more motors. In some cases, the one or more motors may be configured to actuate the one or more brushes. In some embodiments, the brush and hopper subsystem may comprise two or more motors.

In some cases, the brush and hopper subsystem may comprise a hopper assembly. In some cases, the hopper assembly may be attachable to a brush deck assembly of the brush and hopper subsystem.

In some cases, the brush and hopper subsystem may comprise one or more springs. In some cases, the one or more springs may be configured to control or dampen a movement or a motion of the one or more brushes. In some cases, the brush and hopper subsystem may comprise a pulley assembly. In some cases, the pulley assembly may be configured to lift or otherwise change the position of the one or more brushes. In some cases, the brush and hopper subsystem may comprise one or more shoulder bolts. The one or more shoulder bolts may be used to secure the pulley assembly to the motor assembly used to drive the one or more side brushes.

In some cases, the brush and hopper subsystem may comprise one or more actuators. In some cases, the one or more actuators may comprise a linear actuator. The one or more actuators may be configured to move the brush deck up or down. In some cases, the brush and hopper subsystem may comprise a bracket for the one or more actuators.

### Fleet of Robots

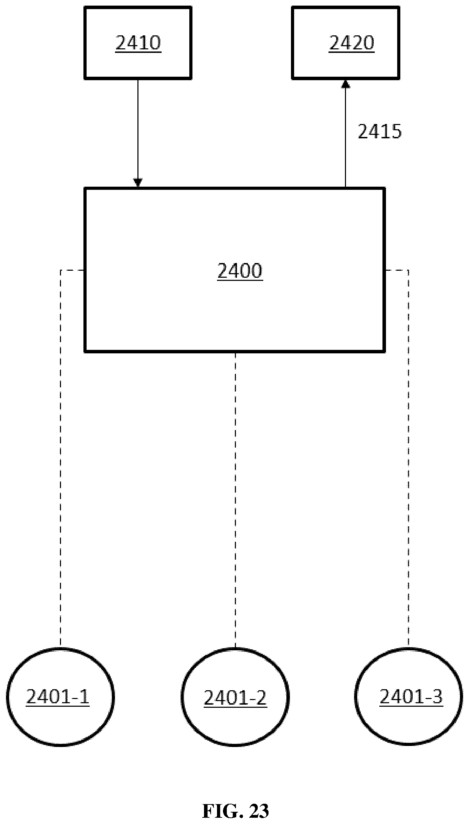

**FIG. 23** schematically illustrates a central server 2400 and a plurality of robots and/or machines 2401-1, 2401-2, and 2401-3 that are in communication with the central server 2400. In some cases, the central server 2400 may be configured to receive operational data from the plurality of robots and/or machines 2401-1, 2401-2, and 2401-3. The plurality of robots and/or machines 2401-1, 2401-2, and 2401-3 may be in communication with each other. Alternatively, the plurality of robots and/or machines 2401-1, 2401-2, and 2401-3 may not or need not be in communication with each other.

The plurality of robots and/or machines 2401-1, 2401-2, and 2401-3 may each comprise one or more sensors. The one or more sensors may be used to capture the operational data associated with the operation or the status of the plurality of robots and/or machines 2401-1, 2401-2, and 2401-3.

The central server 2400 may be configured to process the operational data. In some embodiments, the central server 2400 may be configured to compare the operational data to one or more reference values or thresholds associated with the operation or the status of the one or more robots or machines or one or more components of the one or more robots or machines. In some cases, the central server 2400 may be configured to receive the one or more reference values or thresholds from a memory module 2410. The central server 2400 may be configured to detect one or more changes or deviations in operation or expected behavior for the one or more robots or machines or the one or more components of the one or more robots or machines based at least in part on the comparison of the operational data to the one or more reference values or thresholds. The central server 2400 may be configured to generate one or more reports or update an operational logic for the one or more robots or machines based on the detected changes or deviations, or based on one or more metrics computed using the operational data received from the one or more robots or machines.

In some embodiments, the central server 2400 may be configured to generate and transmit one or more reports 2415 to one or more entities 2420. The one or more entities 2420 may comprise an operator or an administrator of the one or more robots or machines. The one or more reports 2415 may comprise one or more metrics associated with an operation of the one or more robots or machines.

### Platform / iSynergy

In some cases, the systems and methods of the present disclosure may be implemented using a platform for collecting and processing operational data of one or more robots or machines. The operational data of each robot or machine in a fleet may be transmitted to a central server or platform, which may be configured to collect and process the operational data. The operational data (and/or any other information that can be derived from the processing of the operational data) may be transmitted to one or more end user interfaces or portals to facilitate the monitoring and control of robot or machine operation. In some cases, the central server or platform may comprise an IoT platform that synergizes the management of multiple cleaning robots or machines in a fleet based on operational data obtained from one or more robots or machines in the fleet.

In some cases, the platform may comprise a cloud server that is in communication with one or more robots or machines via a wireless communication network. The cloud server may be operatively coupled to a plurality of robots or machines that are configured to operate in an environment. In some cases, the environment may be an indoor environment that supports wireless communications.

In some cases, the cleaning robots or machines may be in communication with a cloud server via a network. The network may permit a transmission of data between (i) a service provider or a cloud server and (ii) the cleaning robots or machines. The service provider or cloud server may be configured to process data received from the robots or machines. The service provider or cloud server may be configured to monitor or control an operation of the robots or machines based on the operational data received from the robots or machines. In some cases, the service provider or cloud server may be configured to provide one or more reports, alerts, and/or notifications to a user or an operator of the robots or machines based on the operational data received from the robots or machines. The one or more notifications may indicate, for example, that a change or deviation in expected robot or machine performance or behavior has been detected, or that a variance in a planned motion logic of the robot or machine has been identified. In some cases, the service provider or cloud server may interface with a mobile application or a web application to facilitate tracking of cleaning, robot or machine operation, and/or the processing of fleet information and/or operational data.

### Scannable Codes

In some embodiments, one or more scannable codes may be used to facilitate a cleaning operation. The one or more scannable codes may be associated with or affixable to one or more objects or surfaces in an environment to be cleaned or navigated. In some cases, the one or more scannable codes may comprise a bar code, a quick response (QR) code, an April tag, a unique identifier, or a serial number.

In some embodiments, the one or more scannable codes may be scanned by the robot or machine. Based on such scanning, the robot or machine may initiate a cleaning procedure or move along a predetermined route.

### Motion Paths

In some cases, the operation of the one or more robots or machines may be adjusted based on the operational data obtained for the one or more robots or machines. In some cases, one or more motion paths or cleaning routines assigned to the one or more robots or machines may be adjusted based on the operational data. In some cases, the operation of the one or more robots or machines may be adjusted based on a detected change or deviation in expected robot or machine behavior or performance.

### Computer Systems

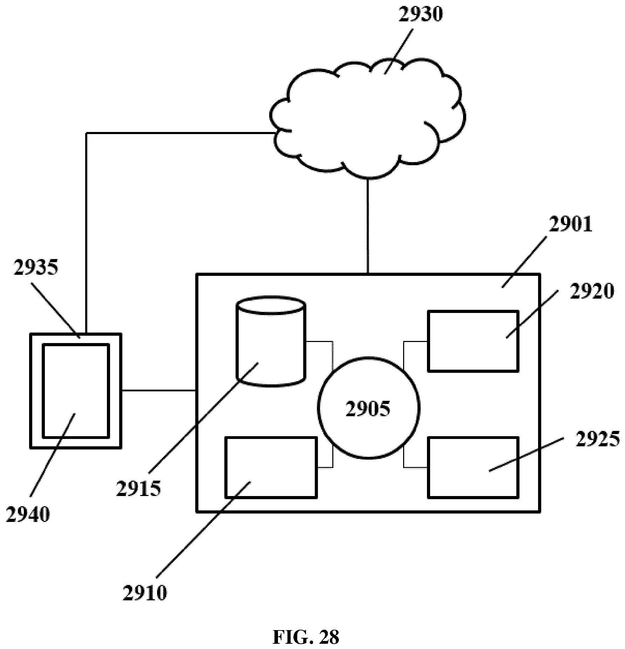

In an aspect, the present disclosure provides computer systems that are programmed or otherwise configured to implement methods of the disclosure, e.g., any of the subject methods for robotic cleaning. Referring to **FIG. 28****,** a computer system 2901 may be programmed or otherwise configured to implement a method for cleaning an environment. The computer system 2901 may be configured to, for example, control a robot to execute a cleaning procedure or operation based on a user input, prior training or teaching, or a decision made by the robot based on sensor readings and/or using artificial intelligence or machine learning. The computer system 2901 can be an electronic device of a user or a computer system that is remotely located with respect to the electronic device. The electronic device can be a mobile electronic device.

The computer system 2901 may include a central processing unit (CPU, also "processor" and "computer processor" herein) 2905, which can be a single core or multi core processor, or a plurality of processors for parallel processing. The computer system 2901 also includes memory or memory location 2910 (e.g., random-access memory, read-only memory, flash memory), electronic storage unit 2915 (e.g., hard disk), communication interface 2920 (e.g., network adapter) for communicating with one or more other systems, and peripheral devices 2925, such as cache, other memory, data storage and/or electronic display adapters. The memory 2910, storage unit2915, interface 2920 and peripheral devices 2925 are in communication with the CPU 2905 through a communication bus (solid lines), such as a moth-erboard. The storage unit 2915 can be a data storage unit (or data repository) for storing data. The computer system 2901 can be operatively coupled to a computer network ("network") 2930 with the aid of the communication interface 2920. The network 2930 can be the Internet, an internet and/or extranet, or an intranet and/or extranet that is in communication with the Internet. The network 2930 in some cases is a telecommunication and/or data network. The network 2930 can include one or more computer servers, which can enable distributed computing, such as cloud computing. The network 2930, in some cases with the aid of the computer system 2901, can implement a peer-to-peer network, which may enable devices coupled to the computer system 2901 to behave as a client or a server.

The CPU 2905 can execute a sequence of machine-readable instructions, which can be embodied in a program or software. The instructions may be stored in a memory location, such as the memory 2910. The instructions can be directed to the CPU 2905, which can subsequently program or otherwise configure the CPU 2905 to implement methods of the present disclosure. Examples of operations performed by the CPU 2905 can include fetch, decode, execute, and writeback.

The CPU 2905 can be part of a circuit, such as an integrated circuit. One or more other components of the system 2901 can be included in the circuit. In some cases, the circuit is an application specific integrated circuit (ASIC).

The storage unit 2915 can store files, such as drivers, libraries and saved programs. The storage unit 2915 can store user data, e.g., user preferences and user programs. The computer system 2901 in some cases can include one or more additional data storage units that are located external to the computer system 2901 (e.g., on a remote server that is in communication with the computer system 2901 through an intranet or the Internet).

The computer system 2901 can communicate with one or more remote computer systems through the network 2930. For instance, the computer system 2901 can communicate with a remote computer system of a user (e.g., an operator or an administrator of a robot or a machine). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 2901 via the network 2930.

Methods as described herein can be implemented by way of machine (e.g., computer processor) executable code stored on an electronic storage location of the computer system 2901, such as, for example, on the memory 2910 or electronic storage unit 2915. The machine executable or machine readable code can be provided in the form of software. During use, the code can be executed by the processor 2905. In some cases, the code can be retrieved from the storage unit 2915 and stored on the memory 2910 for ready access by the processor 2905. In some situations, the electronic storage unit 2915 can be precluded, and machine-executable instructions are stored on memory 2910.

The code can be pre-compiled and configured for use with a machine having a processor adapted to execute the code, or can be compiled during runtime. The code can be supplied in a programming language that can be selected to enable the code to execute in a pre-compiled or as-compiled fashion.

Aspects of the systems and methods provided herein, such as the computer system 2901, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media including, for example, optical or magnetic disks, or any storage devices in any computer(s) or the like, may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 2901 can include or be in communication with an electronic display 2935 that comprises a user interface (UI) 2940 for providing, for example, a portal for a user or an operator to monitor or control an operation of one or more robots or machines. The portal may be provided through an application programming interface (API). A user or entity can also interact with various elements in the portal via the UI. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 2905. For example, the algorithm may be configured to control a robot to execute a cleaning procedure or operation based on a user input, prior training or teaching, or a decision made by the robot based on sensor readings and/or using artificial intelligence or machine learning.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

### THE DISCLOSURE OF THIS APPLICATION ALSO INCLUDES THE FOLLOWING NUMBERED CLAUSES:

1. A floor cleaning machine, comprising:
   a mobile body configured to travel over a surface with aid of a drive mechanism;
   one or more cleaning devices coupled to the mobile body and configured to clean the surface by collecting and removing foreign materials from the surface; and
   a power source carried by the mobile body and configured to power the drive mechanism and the one or more cleaning devices, wherein the power source is configured to enable the floor cleaning machine to operate for a duration ranging from about 0.5 to about 4 hours,
   wherein the floor cleaning machine has a volume ranging from about 0.05 to about 0.30 cubic meters (m³).
2. The floor cleaning machine of clause 1, wherein the floor cleaning machine has a lateral footprint ranging from about 0.10 to 0.40 square meter (m²)
3. The floor cleaning machine of clause 1 or 2, wherein the floor cleaning machine has a weight ranging from about 30 to about 80 kg.
4. The floor cleaning machine of any preceding clause, wherein the drive mechanism is configured to enable a minimum turning radius during cleaning operations of about 500 to about 800 mm for the floor cleaning machine.
5. The floor cleaning machine of any preceding clause, wherein the power source comprises a battery.
6. The floor cleaning machine of clause 5, wherein the battery has a capacity ranging from about 200 Watt-hours to about 900 Watt-hours.
7. The floor cleaning machine of clause 5 or 6, wherein the power source comprises a secondary battery.
8. The floor cleaning machine of clause 7, wherein the secondary battery is detachable from the mobile body.
9. The floor cleaning machine of clause 8, wherein the secondary battery has a capacity ranging from about 200 Watt-hours to about 900 Watt-hours.
10. The floor cleaning machine of any preceding clause, wherein the drive mechanism is configured to enable the floor cleaning machine to move at a speed of up to about 3.6 km/hour.
11. The floor cleaning machine of any preceding clause, further comprising a solution tank to hold a cleaning liquid, wherein the solution tank is carried by the mobile body and has a capacity ranging from about 5L to about 15L.
12. The floor cleaning machine of any preceding clause, further comprising a recovery tank to hold a waste solution collected from the surface being cleaned, wherein the recovery tank is carried by the mobile body and has a capacity ranging from about 5 to about 15L.
13. The floor cleaning machine of any preceding clause, further comprising a hopper to hold the foreign materials collected from the surface, wherein the hopper is carried by the mobile body and has a capacity of up to about 5L.
14. The floor cleaning machine of any preceding clause, wherein the floor cleaning machine is configured to have a cleaning surface productivity rate ranging from about 100 to about 2000 m²/hour
15. The floor cleaning machine of any preceding clause, wherein the one or more cleaning devices comprise a brush, a squeegee, or a mop.
16. The floor cleaning machine of any preceding clause, wherein the one or more cleaning devices are releasably attachable to and detachable from the mobile body.
17. The floor cleaning machine of any preceding clause, wherein the one or more cleaning devices are magnetically attachable to the mobile body.
18. The floor cleaning machine of any preceding clause, wherein the floor cleaning machine is capable of operating at a flow rate ranging from 0 mL/min to about 300 mL/min.
19. The floor cleaning machine of any preceding clause, further comprising a UV light sensor for sanitizing or disinfecting a waste water tank of the floor cleaning machine.
20. The floor cleaning machine of any preceding clause, further comprising a bumper for detecting contact with one or more objects.
21. The floor cleaning machine of clause 20, further comprising a processing unit configured to adjust a movement of the floor cleaning machine based on the detected contact in order to avoid or move around the one or more objects.
22. The floor cleaning machine of any preceding clause, further comprising a pressure adjustment system for the one or more cleaning devices, wherein the pressure adjustment system is configured to adjust a pressure applied to the surface by the one or more cleaning devices by adjusting a position or an orientation of the one or more cleaning devices.
23. The floor cleaning machine of clause 22, wherein the pressure adjustment system is configured to adjust a pressure applied to the surface based on a sensor output.
24. The floor cleaning machine of clause 23, wherein the sensor output is indicative of an amount of dirt, debris, or foreign materials on the surface.
25. The floor cleaning machine of any preceding clause, wherein the floor cleaning machine is configured to receive a premeasured or predetermined dosage of a cleaning compound and to clean the surface using at least a portion of the premeasured or predetermined dosage of the cleaning compound.
26. The floor cleaning machine of any preceding clause, wherein an operational time or duration for the floor cleaning machine is based on a mode of operation or use for the floor cleaning machine.
27. The floor cleaning machine of any preceding clause, wherein the floor cleaning machine comprises a floor scrubber.
28. The floor cleaning machine of any preceding clause, wherein the floor cleaning machine comprises an autonomous or semi-autonomous mobile robot.
29. The floor cleaning machine of any preceding clause, wherein the floor cleaning machine comprises one or more vision sensors and/or one or more navigation sensors.
30. The floor cleaning machine of any preceding clause, wherein the floor cleaning machine comprises a solution tank for storing water and/or a cleaning solution.
31. The floor cleaning machine of any preceding clause, wherein the floor cleaning machine provides an optimal balance of size, weight, operating performance, and run time for cleaning an environment spanning up to about 15,000 square feet or more.
32. The floor cleaning machine of any preceding clause, wherein the floor cleaning machine has a minimum turning radius while cleaning or performing a cleaning operation, wherein the minimum turning radius ranges from about 500 millimeters to about 800 millimeters.
33. The floor cleaning machine of any preceding clause, wherein the floor cleaning machine is configured to turn in place when the floor cleaning machine is not actively engaged in cleaning or performing a cleaning operation.
34. The floor cleaning machine of any preceding clause when dependent upon clause 15, wherein the brush comprises at least one of a rolling brush or a side brush.
35. The floor cleaning machine of clause 34, wherein the side brush comprises long bristles, short bristles, outer bristles, or any combination thereof.
36. The floor cleaning machine of clause 34 or 35, wherein the side brush comprises at least one liquid ejection hole that is configured to dispense cleaning liquid from a solution tank of the floor cleaning machine.
37. The floor cleaning machine of clause 34, 35 or 36, wherein the side brush comprises at least one liquid displacing component configured to displace liquid from the surface toward a predetermined direction or region.
38. The floor cleaning machine of clause 37, wherein the at least one liquid displacing component comprises an elastic blade having an edge that is in contact or close proximity with the surface.
39. The floor cleaning machine of clause 38, wherein the elastic blade extends radially from a center of the side brush.
40. The floor cleaning machine of clause 39, wherein the elastic blade has a shape that is curved based at least on a rotating direction of the side brush.
41. The floor cleaning machine of any of clauses 37 to 40, wherein the width of the squeegee is equal to or slightly greater than the width of the body of the floor cleaning machine.

## Claims

1. A floor cleaning machine, comprising:
a mobile body configured to travel over a surface with aid of a drive mechanism;
one or more cleaning devices operably coupled to the mobile body and configured to clean the surface by collecting and removing foreign materials from the surface; and
a power source carried by the mobile body and configured to power the drive mechanism and the one or more cleaning devices, wherein the power source is configured to enable the floor cleaning machine to operate for a duration ranging from about 0.5 to about 4 hours,
wherein the floor cleaning machine has a volume ranging from about 0.05 to about 0.30 cubic meters (m³).

2. The floor cleaning machine of claim 1, wherein the floor cleaning machine has a lateral footprint ranging from about 0.10 to 0.40 square meter (m²) and/or a weight ranging from about 30 to about 80 kg.

3. The floor cleaning machine of claim 1 or 2, wherein the drive mechanism is configured to enable a minimum turning radius of about 500 to about 800 mm during cleaning operations.

4. The floor cleaning machine of any preceding claim, wherein the power source comprises a battery having a capacity ranging from about 200 Watt-hours to about 900 Watt-hours, optionally wherein the power source further comprises a secondary battery that is detachable from the mobile body, further optionally wherein the secondary battery has a capacity ranging from about 200 Watt-hours to about 900 Watt-hours.

5. The floor cleaning machine of any preceding claim, wherein the drive mechanism is configured to enable the floor cleaning machine to move at a speed of up to about 3.6 km/hour.

6. The floor cleaning machine of any preceding claim, further comprising:
a solution tank to hold a cleaning liquid, wherein the solution tank is carried by the mobile body and has a capacity ranging from about 5L to about 15L; and/or
a recovery tank to hold a waste solution collected from the surface being cleaned, wherein the recovery tank is carried by the mobile body and has a capacity ranging from about 5 to about 15L; and/or
a hopper to hold the foreign materials collected from the surface, wherein the hopper is carried by the mobile body and has a capacity of up to about 5L.

7. The floor cleaning machine of any preceding claim, wherein the floor cleaning machine is configured to have a cleaning surface productivity rate ranging from about 100 to about 2000 m²/hour.

8. The floor cleaning machine of any preceding claim, wherein:
the one or more cleaning devices comprise a brush, a squeegee, or a mop; and/or
the one or more cleaning devices are releasably attachable to and detachable from the mobile body; and/or
the one or more cleaning devices are magnetically attachable to the mobile body.

9. The floor cleaning machine of any preceding claim, wherein the floor cleaning machine is configured to operate at a flow rate ranging from 0 mL/min to about 300 mL/min.

10. The floor cleaning machine of any preceding claim, further comprising an ultra-violet (UV) light sensor for sanitizing or disinfecting a waste-water tank of the floor cleaning machine.

11. The floor cleaning machine of any preceding claim, further comprising a bumper for detecting contact with one or more objects and a processing unit configured to adjust a movement of the floor cleaning machine based on the detected contact in order to avoid or move around the one or more objects.

12. The floor cleaning machine of any preceding claim, further comprising a pressure adjustment system for the one or more cleaning devices, wherein the pressure adjustment system is configured to adjust a pressure applied to the surface by the one or more cleaning devices by adjusting a position or an orientation of the one or more cleaning devices, optionally wherein the pressure adjustment system is configured to adjust a pressure applied to the surface based on a sensor output indicative of an amount of dirt, debris, or foreign materials on the surface.

13. The floor cleaning machine of any preceding claim, wherein the floor cleaning machine is configured to carry a premeasured or predetermined dosage of a cleaning compound and to clean the surface using at least a portion of the premeasured or predetermined dosage of the cleaning compound.

14. The floor cleaning machine of any preceding claim, wherein an operational time or duration for the floor cleaning machine is based on a mode of operation or use for the floor cleaning machine.

15. The floor cleaning machine of any preceding claim, wherein:
the floor cleaning machine comprises a floor scrubber; and/or
the floor cleaning machine comprises an autonomous or semi-autonomous mobile robot; and/or
the floor cleaning machine comprises one or more vision sensors and/or one or more navigation sensors; and/or
the floor cleaning machine is configured to clean an environment spanning up to about 15,000 square feet.
